# EUROPÄISCHE PATENTSCHRIFT

(11) **EP 0 713 488 B1**
(45) Veröffentlichungstag und Bekanntmachung des Hinweises auf die Patenterteilung: **15.10.1997**
(21) Anmeldenummer: 94923712.7
(22) Anmeldetag: 07.07.1994
(51) Int. Cl.: C09B 59/00, C07D 471/04

(54) **[1,3,4]TRIAZOLO[1,5-AL]PYRIDINE ALS ZWISCHENPRODUKTE FÜR DIE SYNTHESE VON FARBSTOFFEN**
[1,3,4]TRIAZOLO [1,5-AL]PYRIDINES USED AS INTERMEDIATE PRODUCTS FOR THE SYNTHESIS OF DYES
[1,3,4]TRIAZOLO[1,5-AL]PYRIDINES UTILISEES COMME INTERMEDIAIRES POUR LA SYNTHESE DE COLORANTS

(30) Priorität: 10.08.1993 DE 4326758
(43) Veröffentlichungstag der Anmeldung: 29.05.1996
(73) Patentinhaber: BASF AKTIENGESELLSCHAFT, 67056 Ludwigshafen (DE)
(72) Erfinder: SCHEFCZIK, Ernst, D-67069 Ludwigshafen (DE)
(86) Internationale Anmeldenummer: EP9402233
(87) Internationale Veröffentlichungsnummer: WO9504733

(56) Entgegenhaltungen:
- EP-A- 0 210 648
- EP-A- 0 441 718
- US-A- 5 101 028
- J.ORG.CHEM. Bd. 35, Nr. 3 , 1970 Seiten 808 - 811 POTTS ET AL '1,2,4-Triazoles.'
- BULL.CHEM.SOC.JAPAN Bd. 53, Nr. 7 , 1980 Seiten 2007 - 11 ITO ET AL 'The preparation of 3-Phenyl[1,2,4]triazolo[4,3-a]pyridines and their Benzologs.'
- J.ORG.CHEM. Bd. 31, Nr. 11 , 1966 Seiten 3522 - 27 POTTS AT EL '1,2,4-triazoles'
- J.CHEM.RESEARCH SYNOP. 1993 Seiten 8 - 9 AYMAN 'Ammonium 1,1,3-Tricyano-2-methylprop-2-enide: A novel reagent in heterocyclic synthesis'

## Beschreibung

Die vorliegende Erfindung betrifft neue Triazolopyridine der Formel I 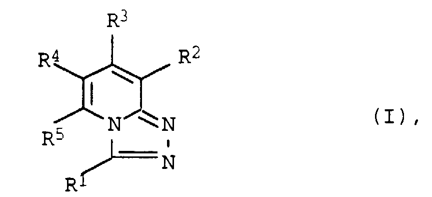 in der
- R¹: C₁-C₂₀-Alkyl, das gegebenenfalls substituiert ist und durch 1 bis 4 Sauerstoffatome in Etherfunktion unterbrochen sein kann, gegebenenfalls durch C₁-C₄-Alkyl, C₁-C₄-Alkoxy, Halogen, Nitro oder Carboxyl substituiertes Phenyl, Mercapto oder gegebenenfalls durch Phenyl, C₁-C₄-Alkylphenyl, C₁-C₄-Alkoxyphenyl, Halogenphenyl, Nitrophenyl, Carboxylphenyl, Phenoxy, C₁-C₄-Alkylphenoxy, C₁-C₄-Alkoxyphenoxy, Halogenphenoxy, Nitrophenoxy, Carboxylphenoxy, Carboxyl oder C₁-C₂₀-Alkoxycarbonyl, dessen Alkylkette gegebenenfalls durch 1 bis 4 Sauerstoffatome in Etherfunktion unterbrochen ist und durch Phenyl oder Phenoxy substituiert sein kann, substituiertes C₁-C₂₀-Alkylthio,
- R²: Wasserstoff, Formyl, Nitroso, Cyano, C₁-C₄-Alkoxymethyl oder einen Rest der Formel CH₂-NL¹L², wobei L¹ und L² gleich oder verschieden sind und unabhängig voneinander jeweils für Wasserstoff oder C₁-C₄-Alkyl, das gegebenenfalls durch C₁-C₄-Alkylimino unterbrochen ist, oder zusammen mit dem sie verbindenden Stickstoffatom für einen 5- oder 6-gliedrigen gesättigten heterocyclischen Rest stehen,
- R³: C₁-C₄-Alkyl, das gegebenenfalls durch ein Sauerstoffatom in Etherfunktion unterbrochen ist, C₁-C₄-Alkoxycarbonyl oder Phenyl,
- R⁴: Wasserstoff, Cyano, Carbamoyl, Carboxyl oder C₁-C₄-Alkoxycarbonyl und
- R⁵: Hydroxy, Mercapto, Halogen, den Rest -NL¹L², wobei L¹ und L² jeweils die obengenannte Bedeutung besitzen, oder den Rest einer CH-aciden Verbindung bedeuten, wobei als CH-acide Verbindungen Nitromethan, Nitroethan oder Verbindungen der Formeln VII bis XII 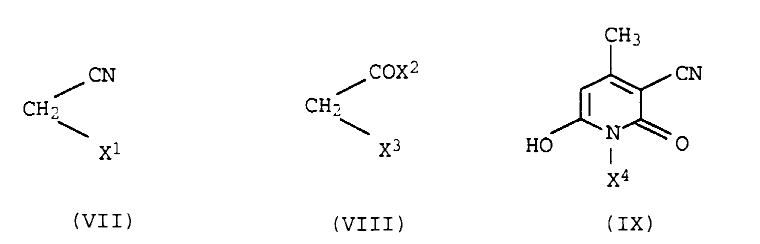 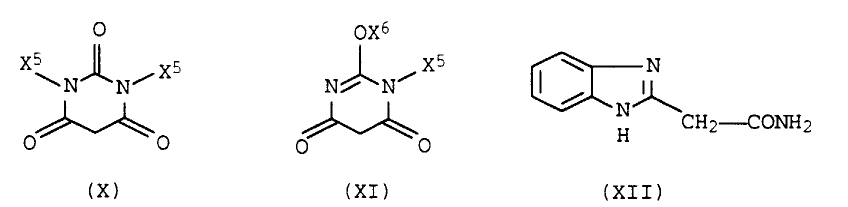
in Betracht kommen, wobei
- X¹: Cyano, Nitro, C₁-C₄-Alkanoyl, gegebenenfalls durch C₁-C₄-Alkyl, C₁-C₄-Alkoxy oder Halogen substituiertes Benzoyl, C₁-C₄-Alkylsulfonyl, gegebenenfalls durch C₁-C₄-Alkyl, C₁-C₄-Alkoxy oder Halogen substituiertes Phenylsulfonyl, Carboxyl, C₁-C₄-Alkoxycarbonyl, Phenoxycarbonyl, Carbamoyl, C₁-C₄-Mono- oder Dialkylcarbamoyl, gegebenenfalls durch C₁-C₄-Alkyl, C₁-C₄-Alkoxy oder Halogen substituiertes Phenylcarbamoyl, gegebenenfalls durch C₁-C₄-Alkyl, C₁-C₄-Alkoxy, Halogen oder Nitro substituiertes Phenyl, Benzthiazol-2-yl, Benzimidazol-2-yl, 5-Phenyl-1,3,4-thiadiazol-2-yl oder 2-Hydroxychinoxalin-3-yl,
- X²: C₁-C₄-Alkyl oder C₁-C₄-Alkoxy,
- X³: C₁-C₄-Alkoxycarbonyl, Phenylcarbamoyl oder Benzimidazol-2-yl,
- X⁴: Wasserstoff oder C₁-C₆-Alkyl,
- X⁵: Wasserstoff, C₁-C₄-Alkyl oder Phenyl und
- X⁶: C₁-C₄-Alkyl bedeuten.
sowie Verfahren zur Herstellung von Triazolopyridinen.

Aus der US-A-5 101 028 sind [1,2,4]Triazolo[1,5-a]pyridine bekannt.

Aufgabe der vorliegenden Erfindung war es nun, neue Triazolopyridine mit anderer chemischer Struktur bereitzustellen. Sie sollten in einfacher Weise herzustellen sein.

Demgemäß wurden die eingangs näher bezeichneten Triazolopyridine der Formel I gefunden.

Die exakte IUPAC-Bezeichnung des Grundkörpers, dem die neuen Triazolopyridine der Formel I zugrundeliegen, lautet [1,3,4]Triazolo[1,5-a]pyridin, wobei die folgende Bezifferung der Ringe gilt:

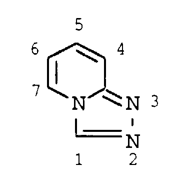

Die Verbindungen der Formel I können in mehreren tautomeren Formen auftreten, die alle von den Patentansprüchen umfaßt werden. Beispielsweise können die Verbindungen mit R⁵ = Hydroxy u.a. in folgenden tautomeren Formen auftreten:

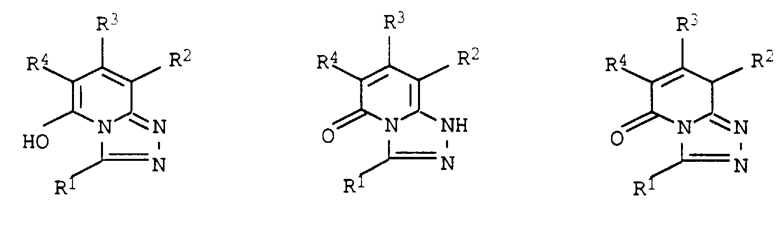

Wenn in Formel I substituierte C₁-C₂₀-Alkylreste auftreten, so weisen sie dabei in der Regel 1 oder 2 Substituenten auf.

Wenn in der Formel I Alkylreste auftreten, die durch Sauerstoffatome in Etherfunktion unterbrochen sind, so sind solche Alkylreste bevorzugt, die durch 1 bis 2 Sauerstoffatome in Etherfunktion unterbrochen sind.

Wenn in der Formel I substituierte Phenylreste auftreten, so weisen sie dabei in der Regel 1 bis 3 Substituenten auf. Halogenphenyl ist insbesondere durch Chlor oder Brom substituiertes Phenyl.

Wenn R⁵ in Formel I einen Rest einer CH-aciden Verbindung bedeutet, so sind insbesondere CH-acide Verbindungen der Formel VII, VIII oder X zu nennen, wobei
- X¹: Cyano, Acetyl, Benzoyl, C₁-C₄-Alkoxycarbonyl, Phenoxycarbonyl, C₁-C₂-Monoalkylcarbonyl, Phenylcarbamoyl, Phenyl, Benzimidazol-2-yl, Benzthiazol-2-yl oder 5-Phenyl-1,3,4-thiazol-2-yl,
- X²: C₁-C₂-Alkoxy,
- X³: C₁-C₂-Alkoxycarbonyl oder Phenylcarbamoyl und
- X⁵: Methyl
bedeuten.

Reste R¹ und R³ sind z.B. Methyl, Ethyl, Propyl, Isopropyl, Butyl, Isobutyl oder sec-Butyl.

Reste R¹ sind weiterhin z.B. Pentyl, Isopentyl, Neopentyl, tert-Pentyl, Hexyl, Heptyl, 1-Ethylpentyl, Octyl, Isooctyl, 2-Ethylhexyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl, Eicosyl (Die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Enzyklopädie der technischen Chemie, 4. Auflage, Band 7, Seiten 215 bis 217, sowie Band 11, Seiten 435 und 436.), 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 3,6-Dioxahepyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- oder 4-Butoxybutyl, 4,8-Dioxadecyl, 3,6,9-Trioxadecyl, 3,6,9-Trioxaundecyl, 3,6,9,12-Tetraoxatridecyl, 3,6,9-Trioxatetradecyl, 2-Carboxyethyl, 2-Methoxycarbonylethyl, Benzyl, 1- oder 2-Phenylethyl, 2-, 3- oder 4-Methylbenzyl, 2-, 3- oder 4-Methoxybenzyl, 2-, 3-oder 4-Chlorbenzyl, 2-, 3- oder 4-Nitrobenzyl, 3-Benzyloxypropyl, Phenoxymethyl, 6-Phenoxy-4-oxahexyl, 8-Phenoxy-4-oxaoctyl, 2-, 3-oder 4-Methylphenyl, 2-, 3- oder 4-Methoxyphenyl, 2-, 3- oder 4-Chlorphenyl, 2-, 3- oder 4-Nitrophenyl, 2-, 3- oder 4-Carboxyphenyl, Methylthio, Ethylthio, Propylthio, Isopropylthio, Butylthio, Isobutylthio, sec-Butylthio, Pentylthio, Isopentylthio, Neopentylthio, tert-Pentylthio, Hexylthio, Heptylthio, 1-Ethylpentylthio, Octylthio, Isooctylthio, 2-Ethylhexylthio, Nonylthio, Isononylthio, Decylthio, Isodecylthio, Undecylthio, Dodecylthio, Tridecylthio, Isotridecylthio, Tetradecylthio, Pentadecylthio, Hexadecylthio, Heptadecylthio, Octadecylthio, Nonadecylthio oder Eicosylthio.

Reste R⁴ sind z.B. Methoxycarbonyl, Ethoxycarbonyl, Propoxycarbonyl, Isopropoxycarbonyl, Butoxycarbonyl, Isobutoxycarbonyl oder sec-Butoxycarbonyl.

Reste R² sind z.B. Aminomethyl, N-Mono- oder N,N-Dimethylaminomethyl, N-Mono- oder N,N-Diethylaminomethyl, N-Mono- oder N,N-Dipropylaminomethyl, Pyrrolidinomethyl, Piperidinomethyl, Morpholinomethyl, Piperazinomethyl, N-(C₁-C₄-Alkyl)piperazinomethyl, Methoxymethyl, Ethoxymethyl, Propoxymethyl, Isopropoxymethyl oder Butoxymethyl.

Reste R³ sind weiterhin z.B. Methoxymethyl, Ethoxymethyl, 2-Methoxyethyl, 2-Ethoxyethyl, Methoxycarbonyl, Ethoxycarbonyl, Propoxycarbonyl oder Butoxycarbonyl.

Reste R⁵ sind z.B. Amino, Mono- oder Dimethylamino, Mono- oder Diethylamino, Mono- oder Dipropylamino, Mono- oder Diisopropylamino, Mono- oder Dibutylamino, 2-Dimethylaminoethylamino, 2-oder 3-Dimethylaminopropylamino, Pyrrolidino, Piperidino, Morpholino, Piperazino oder N-(C₁-C₄-Alkyl)piperazino.

Bevorzugt sind Triazolopyridine der Formel I, in der einer der beiden Reste R² und R⁴ Wasserstoff und der andere Cyano bedeutet.

Weiterhin bevorzugt sind Triazolopyridine der Formel I, in der R² Cyano bedeutet.

Weiterhin bevorzugt sind Triazolopyridine der Formel I, in der R³ C₁-C₄-Alkyl, insbesondere Methyl bedeutet.

Weiterhin bevorzugt sind Triazolopyridine der Formel I, in der R¹ C₁-C₁₃-Alkyl oder Phenyl bedeutet.

Besonders bevorzugt sind Triazolopyridine der Formel I, in der R² Cyano und R⁴ Wasserstoff bedeuten.

Eine weitere Aufgabe der vorliegenden Erfindung war es, geeignete Verfahren bereitzustellen, mittels denen es gelingen sollte, spezielle Triazolopyridine der Formel I auf einfachem Weg und ohne großen Aufwand herzustellen.

Es wurde weiterhin gefunden, daß die Herstellung der Triazolopyridine der Formel Ia 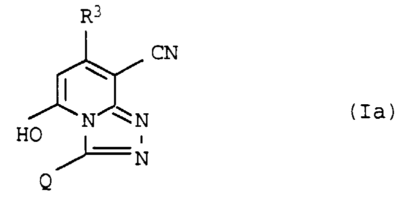 gehorchen, in der
in der Q C₁-C₂₀-Alkyl, das gegebenenfalls durch Phenyl, C₁-C₄-Alkylphenyl, C₁-C₄-Alkoxyphenyl, Halogenphenyl, Nitrophenyl, Carboxylphenyl, Phenoxy, C₁-C₄-Alkylphenoxy, C₁-C₄-Alkoxyphenoxy, Halogenphenoxy, Nitrophenoxy, Carboxylphenoxy, Carboxyl oder C₁-C₂₀-Alkoxycarbonyl, dessen Alkylkette gegebenenfalls durch 1 bis 4 Sauerstoffatome in Etherfunktion unterbrochen ist und durch Phenyl oder Phenoxy substituiert sein kann, substituiert ist und durch 1 bis 4 Sauerstoffatome in Etherfunktion unterbrochen sein kann, oder gegebenenfalls durch C₁-C₄-Alkyl, C₁-C₄-Alkoxy, Halogen, Nitro oder Carboxyl substituiertes Phenyl bedeutet und R³ die obengenannte Bedeutung besitzt, vorteilhaft gelingt, wenn man eine Dicarbonylverbindung der Formel II

R³-CO-CH₂-COOW (II),

in der W C₁-C₄-Alkyl bedeutet und R³ die obengenannte Bedeutung besitzt, mit einem Cyanomethyltriazol der Formel III 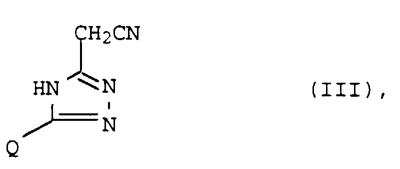 in der Q die obengenannte Bedeutung besitzt, in Gegenwart einer Base und eines Lösungsmittels bei einer Temperatur von 50 bis 150°C umsetzt.

Geeignete Basen sind z.B. Alkalialkanolate, wie Lithium-, Natrium- oder Kaliummethanolat oder -ethanolat, Alkaliamide, wie Lithium-, Natrium- oder Kaliumamid, Alkalihydride, wie Lithium-, Natrium- oder Kaliumhydrid, oder Alkalihydroxide, wie Lithium-, Natrium- oder Kaliumhydroxid.

Geeignete Lösungsmittel sind z.B. Alkohole, wie Methanol, Ethanol, Propanol, Isopropanol, Butanol, Ethylenglykol, Diethylenglykol oder Ethylenglykolmonomethyl- oder -ethylether, oder N-Methylpyrrolidon.

Das Molverhältnis Dicarbonylverbindung II: Cyanomethyltriazol III beträgt im allgemeinen 1:1 bis 3:1, vorzugsweise 1:1 bis 1,5:1.

Pro mol Dicarbonylverbindung II wendet man üblicherweise 1 bis 2 mol Base an.

Nach beendeter Umsetzung, die in der Regel 1 bis 8 Stunden in Anspruch nimmt, wird das Reaktionsgemisch mit Wasser verdünnt und durch Zugabe von Säure, z.B. konzentrierte Salzsäure neutralisiert. Das dabei als Niederschlag anfallende Zielprodukt der Formel Ia kann dann abgetrennt und gegebenenfalls weiter gereinigt werden.

Es wurde weiterhin gefunden, daß die Herstellung der Triazolopyridine der Formel Ib 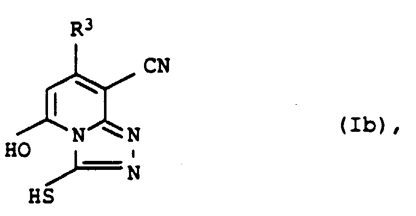 in der R³ die obengenannte Bedeutung besitzt, vorteilhaft gelingt, wenn man ein Hydrazinopyridin der Formel IV 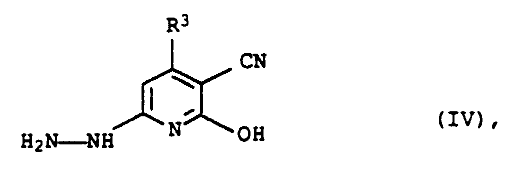 in der R³ die obengenannte Bedeutung besitzt, mit Schwefelkohlenstoff behandelt.

Die Behandlung findet in der Regel bei einer Temperatur von 25 bis 100°C in Gegenwart von Pyridin statt. Pyridin dient dabei als Base und als Verdünnungsmittel. Es ist aber auch möglich, die Umsetzung in einem Alkohol, z.B. Methanol oder Ethanol, in Gegenwart einer Base, z.B. Lithium-, Natrium- oder Kaliummethanolat oder -ethanolat, vorzunehmen.

Je mol Hydrazinopyridin IV wendet man in der Regel 1 bis 10 mol, vorzugsweise 1 bis 5 mol, Schwefelkohlenstoff an.

Je Gewichtsteil Hydrazinopyridin IV kommen im allgemeinen 1 bis 10 Gewichtsteilen Pyridin zur Anwendung.

Nach beendeter Umsetzung, die in der Regel 2 bis 8 Stunden in Anspruch nimmt, wird das entstandene Reaktionsgemisch mit einem Verdünnungsmittel, z.B. Methanol, versetzt und das als Niederschlag anfallende Zielprodukt der Formel Ib isoliert und gegebenenfalls weiter gereinigt.

Es wurde weiterhin gefunden, daß die Herstellung von Triazolopyridinen der Formel Ic 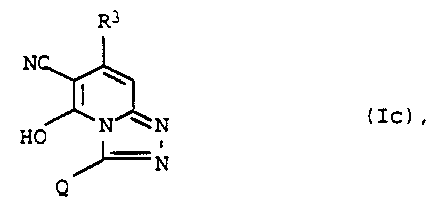 in der Q und R³ jeweils die obengenannte Bedeutung besitzen, vorteilhaft gelingt, wenn man ein Hydrazinopyridin der obengenannten Formel IV mit einem Carbonsaurederivat der Formel V

Q-CO-Y (V)

in der Y Chlor, Brom oder der Rest O-CO-Q bedeutet und Q jeweils die obengenannte Bedeutung besitzt, acyliert und die resultierende Acylverbindung der Formel VI 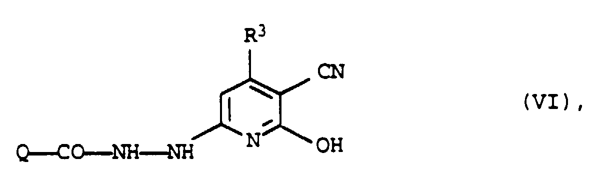 in der Q und R³ jeweils die obengenannte Bedeutung besitzen, unter Ringschluß dehydratisiert.

Die Acylierung findet in der Regel bei einer Temperatur von 25 bis 125°C und in Gegenwart eines Lösungsmittels, z.B. Pyridin oder Chinolin, statt.

Je mol Hydrazinpyridin IV wendet man in der Regel 1 bis 2 mol Carbonsäurederivat V an.

Die resultierende Acylverbindung VI kann entweder zunächst zwischenisoliert werden oder direkt in das Zielprodukt der Formel Ic übergeführt werden.

Die Dehydratisierung findet in der Regel bei einer Temperatur von 80 bis 150°C und in Gegenwart eines Lösungsmittel, z.B. N-Methylpyrrolidon, N,N-Dimethylformamid oder N,N-Dimethylacetamid, sowie eines Dehydratisierungsmittels statt.

Ein geeignetes Dehydratisierungsmittel ist z.B. Phosphorpentoxid.

Je mol Hydrazinopyridin IV verwendet man im allgemeinen 1 bis 1,5 mol Dehydratisierungsmittel.

Nach beendeter Umsetzung, die im allgemeinen 0,5 bis 4 Stunden in Anspruch nimmt, wird das Reaktionsgemisch mit Wasser versetzt. Das Zielprodukt der Formel Ic, das als Niederschlag vorliegt, kann dann abgetrennt und gegebenenfalls weiter gereinigt werden.

Die übrigen Triazolopyridine der Formel I können aus den Verbindungen der Formel Ia, Ib oder Ic auf an sich bekanntem Wege hergestellt werden.

Beispielsweise kann der Substituent R² mittels einer elektrophilen Substitutionsreaktion, z.B. Vilsmeier-Reaktion, oder Nitrosierung, gegebenenfalls mit anschließender Derivatisierung, eingeführt werden.

Die Cyanogruppe in Position 4 oder 6 läßt sich nach an sich bekannten Methoden in die Carbamoyl-, Carboxyl- oder C₁-C₄-Alkoxycarbonylgruppe überführen.

Durch Behandlung mit Saurehalogeniden, insbesondere Säurechloriden, z.B. Phosphoroxidtrichlorid, kann die Hydroxygruppe in Position 7 durch Halogen ersetzt werden.

Das Halogenatom wiederum läßt sich nach an sich bekannten Methoden durch CH-acide Verbindungen, Amine der Formel VII 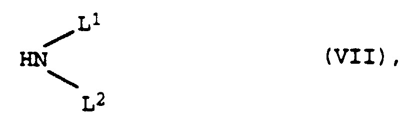 in der L¹ und L² jeweils die obengenannte Bedeutung besitzen, oder Schwefelwasserstoff austauschen.

Die Mercaptoverbindungen der Formel Ib können durch Behandlung mit entsprechenden Alkylierungsmitteln in die jeweiligen Alkylthioverbindungen übergeführt werden.

Die neuen Triazolopyridine der Formel I sind wertvolle Zwischenprodukte, insbesondere für die Synthese von Farbstoffen.

Die folgenden Beispiele sollen die Erfindung näher erläutern.

### Beispiel 1

150 g 3-Isopropyl-5-cyanomethyl-1,2,4-triazol wurden in 250 g Acetessigsäureethylester eingetragen und am absteigenden Kühler bei 75°C gerührt. Man ließ 200 g 30 gew.-%ige methanolische Natriummethanolatlösung zulaufen und erhöhte die Temperatur auf 115°C. Dabei destillierte Methanol ab und das Reaktionsgemisch verwandelte sich in einen Kristallbrei. Nach 6 Stunden gab man 500 ml Wasser und 200 ml konz. Salzsäure zu, kochte kurz auf und ließ erkalten. Dann wurde das ausgefallene Produkt abgesaugt, mit Wasser neutral gewaschen und unter vermindertem Druck bei 75°C getrocknet. Man erhielt 211 g einer farblosen Verbindung der Formel 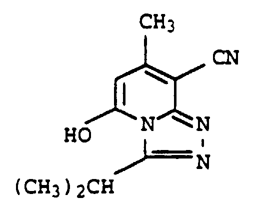 Schmelzpunkt 330 bis 331°C (aus γ-Butyrolacton)

| Analysen C₁₁H₁₂N₄O (216) | | | | |
|---|---|---|---|---|
| ber | C 61,1 | H 5,6 | N 25,9 | O 7,4 |
| gef | C 61,0 | H 5,8 | N 25,6 | O 7,8 |

In analoger Weise werden die in der folgenden Tabelle 1 aufgeführten Triazolopyridine der Formel 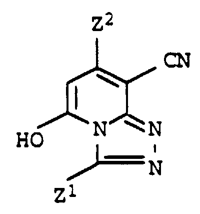 erhalten.

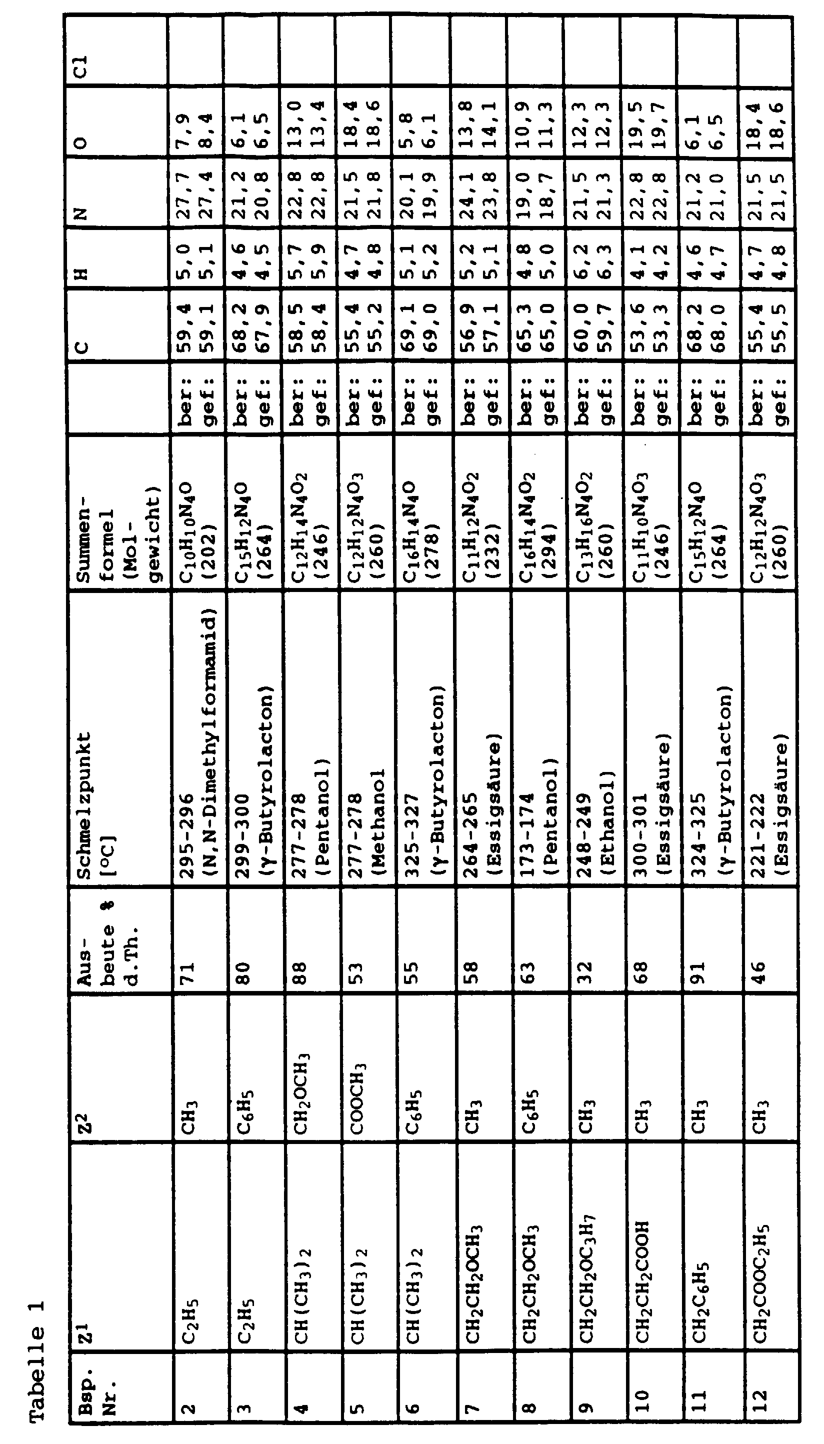

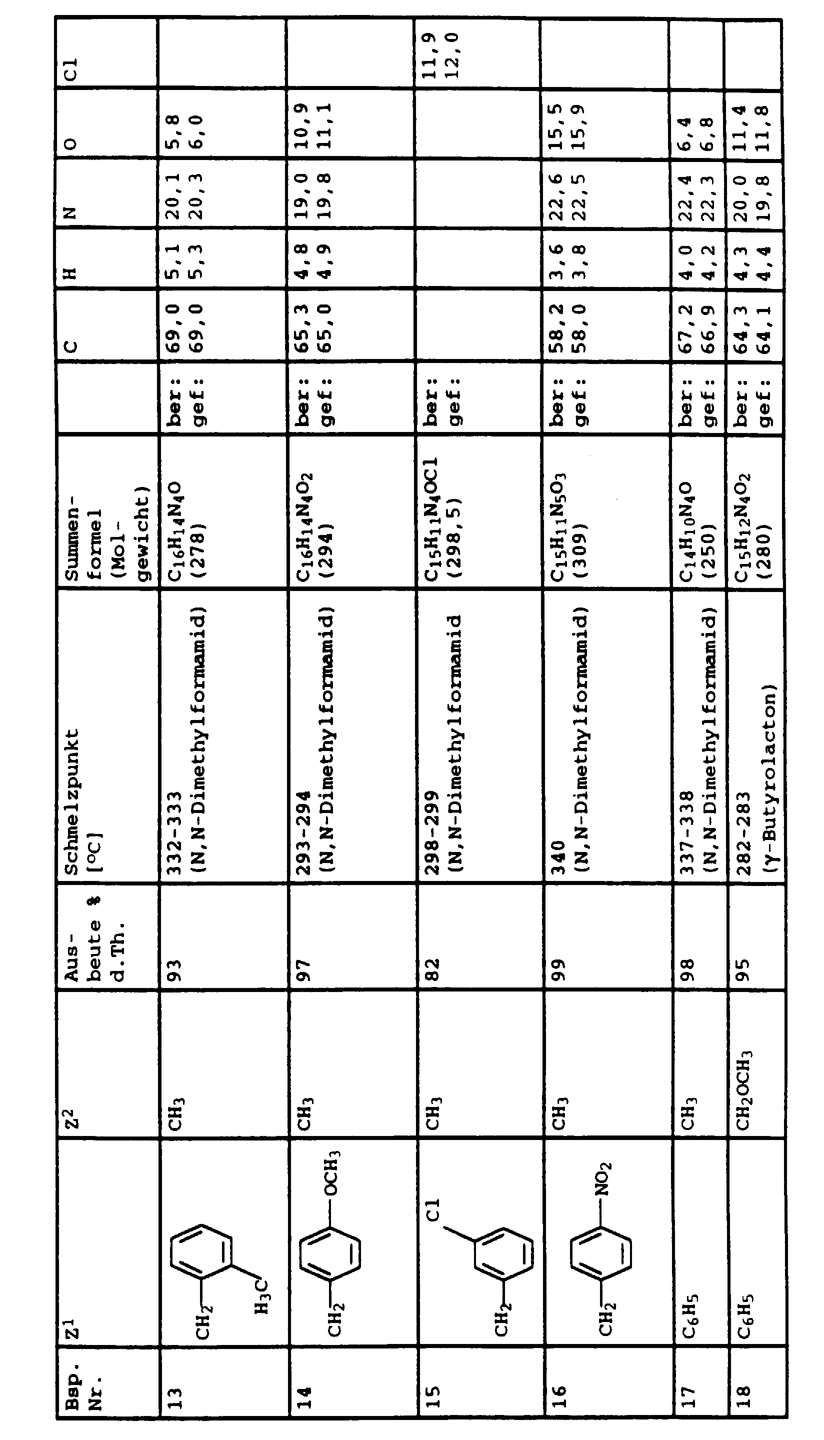

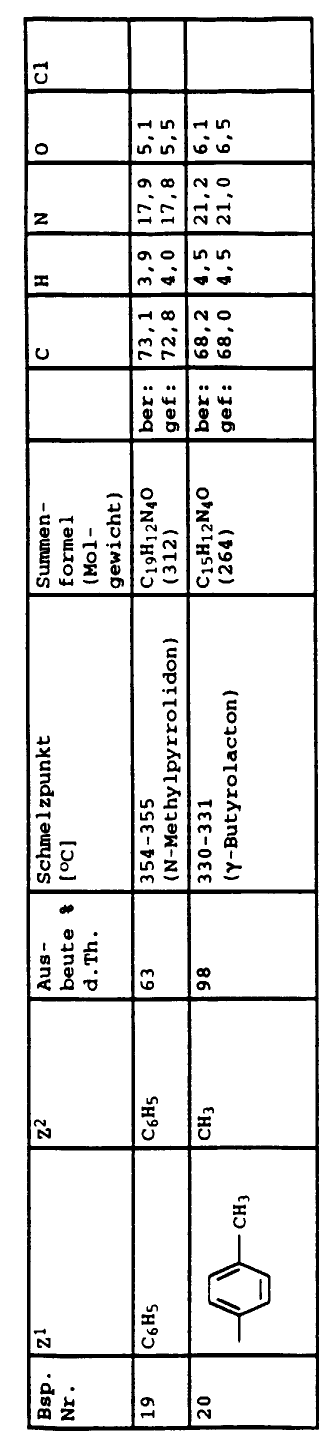

### Beispiel 21

In 80 ml konz. Schwefelsäure wurden 20,2 g der nach Beispiel 2 erhaltenen Verbindung eingetragen und 4 Stunden bei 75°C gerührt. Man fallte auf Eis, saugte ab und wusch mit 5 gew.-%iger Salzsäure nach. Nach dem Trocknen erhielt man 16,8 g der Verbindung der Formel 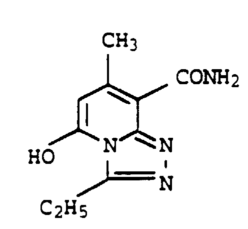 mit einem Schmelzpunkt von 257 bis 258°C (aus γ-Butyrolacton). Das IR-Spektrum zeigt keine Nitrilbande.

### Beispiel 22

In 1500 ml Chloroform und 120 g N,N-Dimethylformamid trug man 250 g der nach Beispiel 17 erhaltenen Verbindung ein und tropfte anschließend unter Rühren 250 g Phosphoroxidtrichlorid zu. Dabei erwärmte sich das Gemisch zum Sieden. Es wurde noch 6 Stunden unter Rückfluß erhitzt, mit 500 ml Wasser versetzt und das Lösungsmittel mit Wasserdampf abdestilliert. Die verbleibende Suspension wurde abgesaugt, das Produkt mit Wasser gewaschen und bei 100°C getrocknet. Man erhielt 268 g der Verbindung der Formel 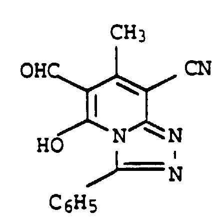 Eine aus N,N-Dimethylformamid umkristallisierte Probe schmilzt bei 274 bis 276°C und zeigt folgende Analyse C₁₅H₁₀N₄O₂ (278)

| | | | | |
|---|---|---|---|---|
| ber | C 64,7 | H 3,6 | N 20,1 | O 11,5 |
| gef | C 64,4 | H 3,7 | N 19,8 | O 11,9 |

Analog Beispiel 22 werden die in der folgenden Tabelle 2 aufgeführten Verbindungen der Formel 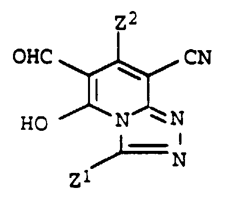 erhalten.

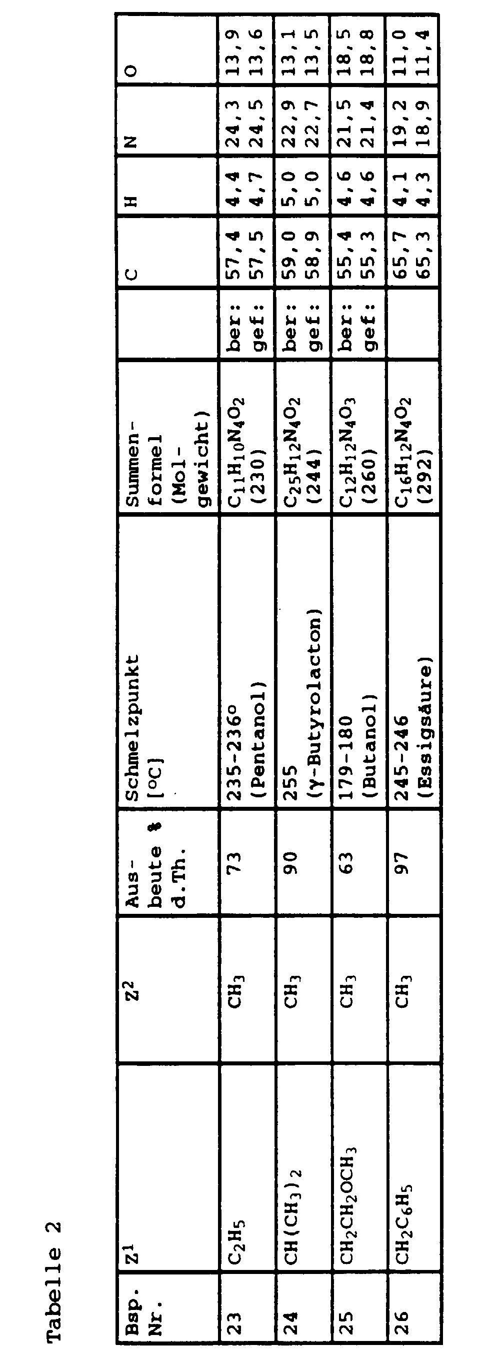

### Beispiel 27

In 600 g Phosphoroxidtrichlorid wurden 250 g der nach Beispiel 17 erhaltenen Verbindung eingetragen und 6 Stunden unter Rückfluß erhitzt. Dann wurde überschüssiges Phosphoroxidtrichlorid unter vermindertem Druck abgezogen, zum Rückstand 1000 ml Ethanol zugetropft und 1 Stunde erhitzt. Nach dem Erkalten wurde abgesaugt, mit Methanol gewaschen und unter vermindertem Druck bei 50°C getrocknet. Man erhielt 247 g der Verbindung der Formel 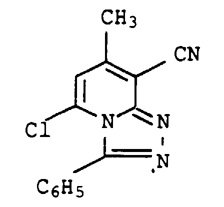 in Form farbloser Kristalle vom Schmelzpunkt 223 bis 224°C (aus Essigsäure).
C₁₄H₉ClN₄ (268,5) ber. Cl 13,2; gef. 13,3

Analog Beispiel 27 werden die in der folgenden Tabelle 3 aufgeführten Verbindungen der Formel 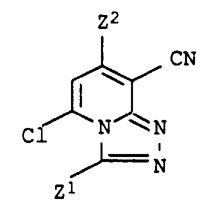 erhalten.

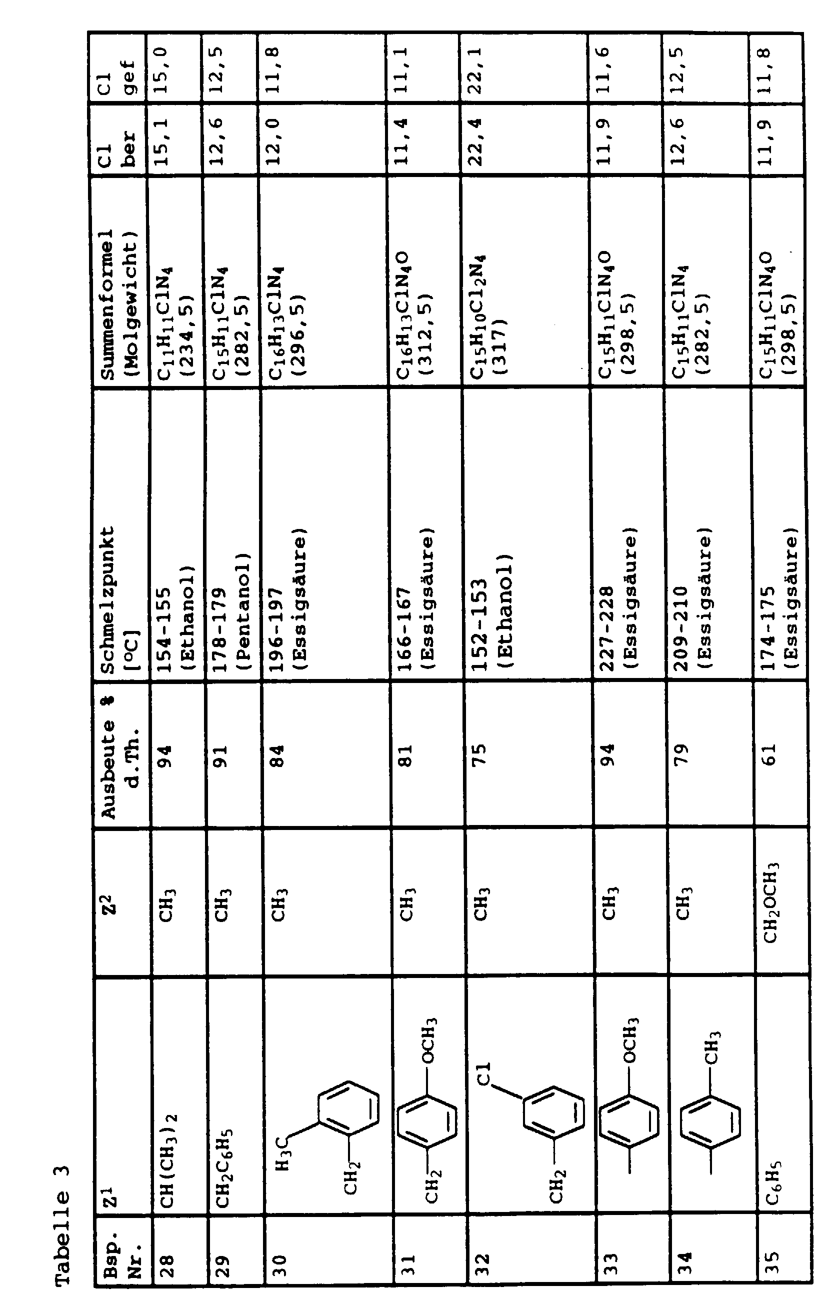

### Beispiel 36

In 500 g Phosphoroxidtrichlorid wurden 232 g der nach Beispiel 7 erhaltenen Verbindung eingetragen und 6 Stunden unter Rückfluß erhitzt. Nach Aufarbeitung, wie in Beispiel 27 beschrieben, erhielt man 193 g der Verbindung der Formel 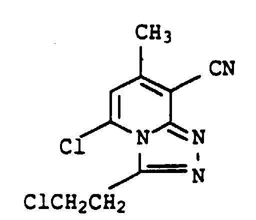 mit einem Schmelzpunkt von 135 bis 136°C (aus Ethanol). C₁₀H₈Cl₂N₄ (255) ber. Cl 27,8, gef. Cl 27,6

### Beispiel 37

In einer Mischung aus 1000 ml Ethanol, 200 g Diethylamin und 300 ml Wasser wurden 234,5 g der nach Beispiel 28 erhaltenen Verbindung eingetragen und 4 Stunden unter Rückfluß erhitzt. Dann destillierte man die Hälfte des Lösungsmittels ab, versetzte mit 500 ml Wasser und ließ erkalten, dabei kristallisierte das Reaktionsprodukt aus. Nach dem Absaugen und Trocknen bei 50°C unter vermindertem Druck erhielt man 236 g der Verbindung der Formel 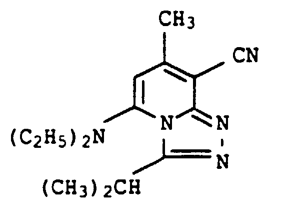 mit einem Schmelzpunkt von 94 bis 95°C (aus Cyclohexan).

| C₁₅H₂₁N₅ (271) | | | |
|---|---|---|---|
| ber | C 66,4 | H 7,8 | N 25,8 |
| gef | C 66,5 | H 7,9 | N 25,9 |

Analog Beispiel 37 werden die in der folgenden Tabelle 4 aufgeführten Verbindungen der Formel 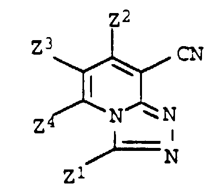 erhalten.

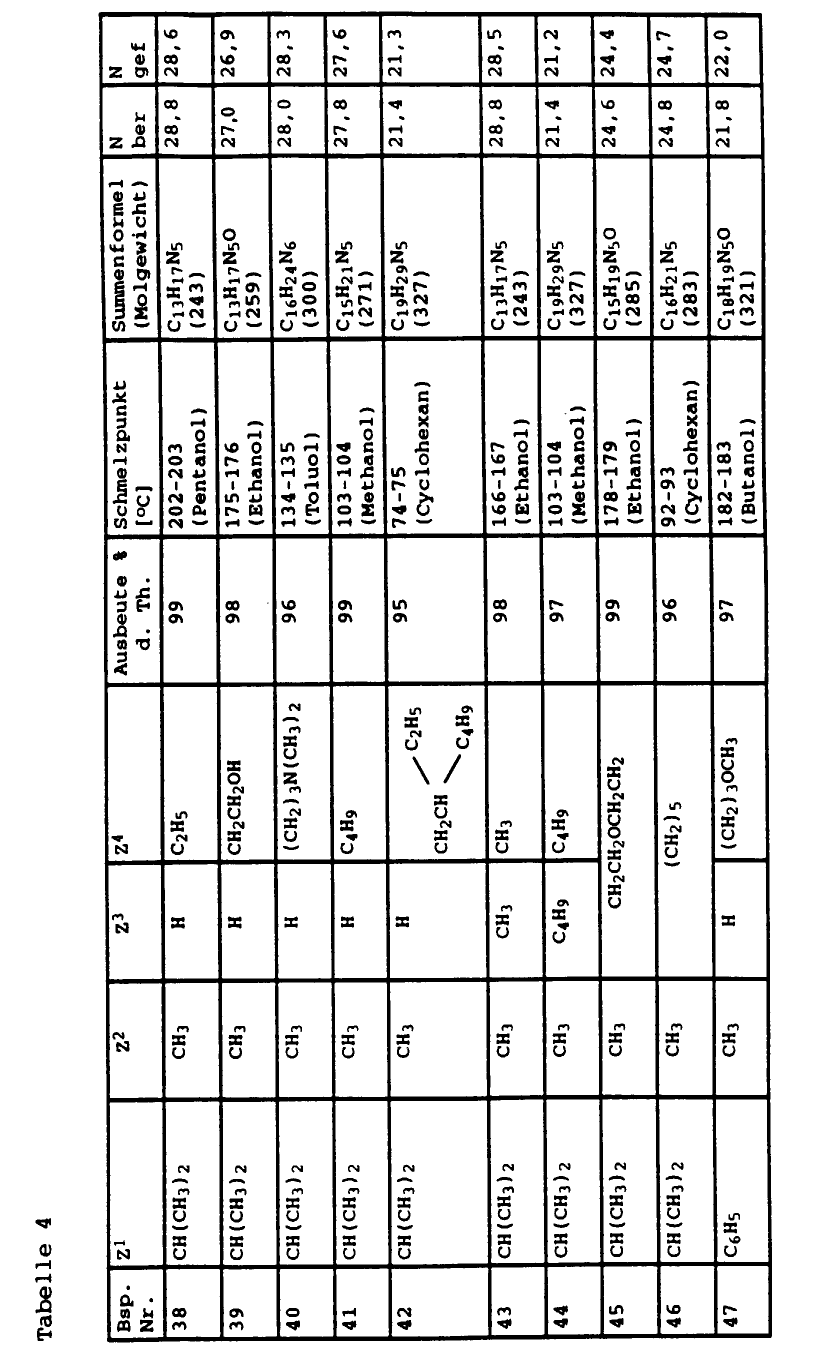

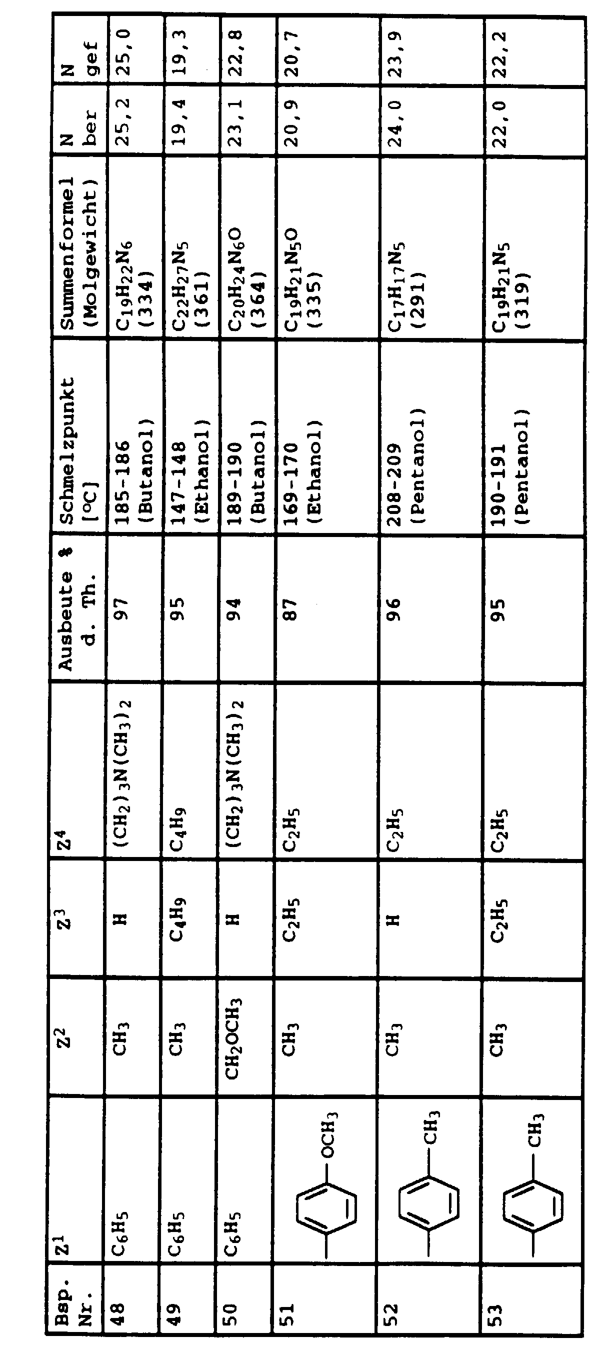

### Beispiel 54

In eine Lösung von 75 g Natriummethanolat in 2000 ml Methanol wurden 268,5 g der nach Beispiel 27 erhaltenen Verbindung eingetragen und 8 Stunden unter Rückfluß erhitzt. Der Ansatz wurde dann auf Wasser gegossen, die ausgefallene Verbindung ausgesaugt und mit Wasser gewaschen. Man erhielt nach dem Trocknen 260 g der Verbindung der Formel 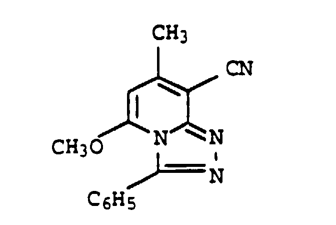 mit einem Schmelzpunkt von 230 bis 231°C (aus Essigsaure).

### Beispiel 55

In 1500 ml 1-Methoxypropan-2-ol wurden 120 g Triethylamin und 234,5 g der nach Beispiel 28 erhaltenen Verbindung eingetragen. Man leitete gasförmigen Schwefelwasserstoff in das Gemisch und steigern die Temperatur innerhalb einer Stunde auf 100°C. Nach 4 Stunden wurde der Ansatz auf Wasser und überschüssiger Salzsäure gefällt und das Produkt abgesaugt. Nach Waschen mit Wasser und Trocknen erhielt man 218,8 g der Verbindung der Formel 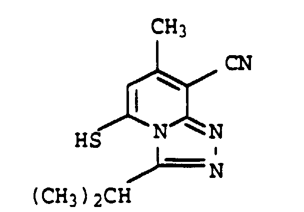 mit einem Schmelzpunkt von 254 bis 255°C (aus γ-Butyrolacton/ Essigsäure).

| C₁₁H₁₂N₄S (232) | | | | |
|---|---|---|---|---|
| ber | C 56,9 | H 5,2 | N 24,1 | S 13,8 |
| gef | C 57,0 | H 5,2 | N 24,0 | S 13,7 |

### Beispiel 56

In 1500 ml abs. Ethanol wurden 130 g Mercaptoessigsäureethylester und 234,5 g der nach Beispiel 28 erhaltenen Verbindung eingetragen und bei Raumtemperatur gerührt. Dazu ließ man 120 g Triethylamin zulaufen, wobei die Temperatur auf 50°C anstieg. Man erhitzte 2 Stunden unter Rückfluß und ließ die klare Lösung erkalten. Dabei kristallisierte das Reaktionsprodukt aus. Die Kristallisation wurde durch Zugabe von 1200 ml Wasser vervollständigt, das Reaktionsprodukt abgesaugt, mit Wasser gewaschen und unter vermindertem Druck bei 70°C getrocknet. Man erhielt 302 g farblose Kristalle der Verbindung der Formel 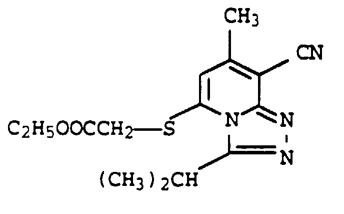 vom Schmelzpunkt 139 bis 140°C (aus Ethanol).
C₁₅H₁₈N₄O₂S (318) : ber S 10,0, gef S 9,8

### Beispiel 57

Man ersetzte in Beispiel 56 Mercaptoessigsäureethylester durch 90 g 2-Mercaptoethanol und verfuhr ansonsten, wie in Beispiel 56 beschrieben. Man erhielt 167 g der Verbindung der Formel 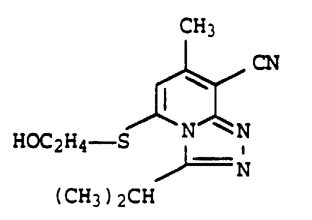 mit einem Schmelzpunkt von 166 bis 167°C (aus Propanol).
C₁₅H₁₆N₄OS (276): ber S 11,6, gef S 11,5

### Beispiel 58

15 g Malodinitril wurden in 200 ml N,N-Dimethylformamid bei einer Temperatur unterhalb von 20°C mit 25 g Triethylamin versetzt. Dazu gab man 23,5 g der nach Beispiel 28 erhaltenen Verbindung. Man rührte 6 Stunden bei 90°C, goß den Ansatz auf 400 ml Wasser und stellte durch Zugabe von Salzsäure auf einen pH-Wert von 3. Dann wurde das ausgefallene Reaktionsprodukt abgesaugt, mit Wasser gewaschen und bei 100°C getrocknet. Man erhielt 25,1 g der Verbindung der Formel 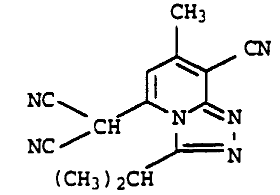 mit einem Schmelzpunkt von 328 bis 330°C (aus N,N-Dimethylformamid/Essigsäure).

| C₁₄H₁₂N₆ (264) | | | |
|---|---|---|---|
| ber | C 63,6 | H 4,6 | N 31,8 |
| gef | C 63,4 | H 4,8 | N 31,5 |

### Beispiel 59

164 g 2-Hydroxy-3-cyano-4-methyl-6-hydrazinopyridin wurden in 1000 ml Pyridin bei 70°C gerührt und mit 192 g der Verbindung der Formel (CH₃)₃C(CH₂)₅COCl versetzt. Man erhitzte 6 Stunden unter Rückfluß und goß die Lösung auf 3000 g Eis und 1200 g konz. Salzsäure. Das ausgefallene Produkt wurde abgesaugt, mit Wasser gewaschen und bei 100°C getrocknet. Man erhielt 288 g der Verbindung der Formel 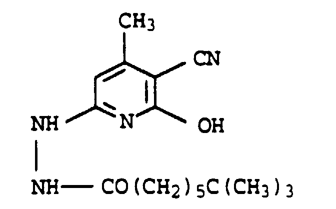 Eine aus Ethanol umkristallisierte Probe schmilzt bis 285 bis 286°C und zeigt folgende Analysenwerte:

| C₁₇H₂₆N₄O₂ (318) | | | | |
|---|---|---|---|---|
| ber | C 64,1 | H 8,2 | N 17,6 | O 10,0 |
| gef | C 64,0 | H 8,4 | N 17,6 | O 10,0 |

Analog Beispiel 59 werden die in der folgenden Tabelle 5 aufgeführten Verbindungen der Formel 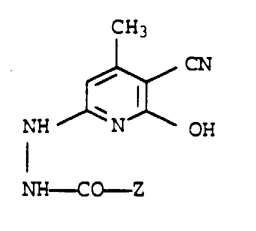 erhalten.

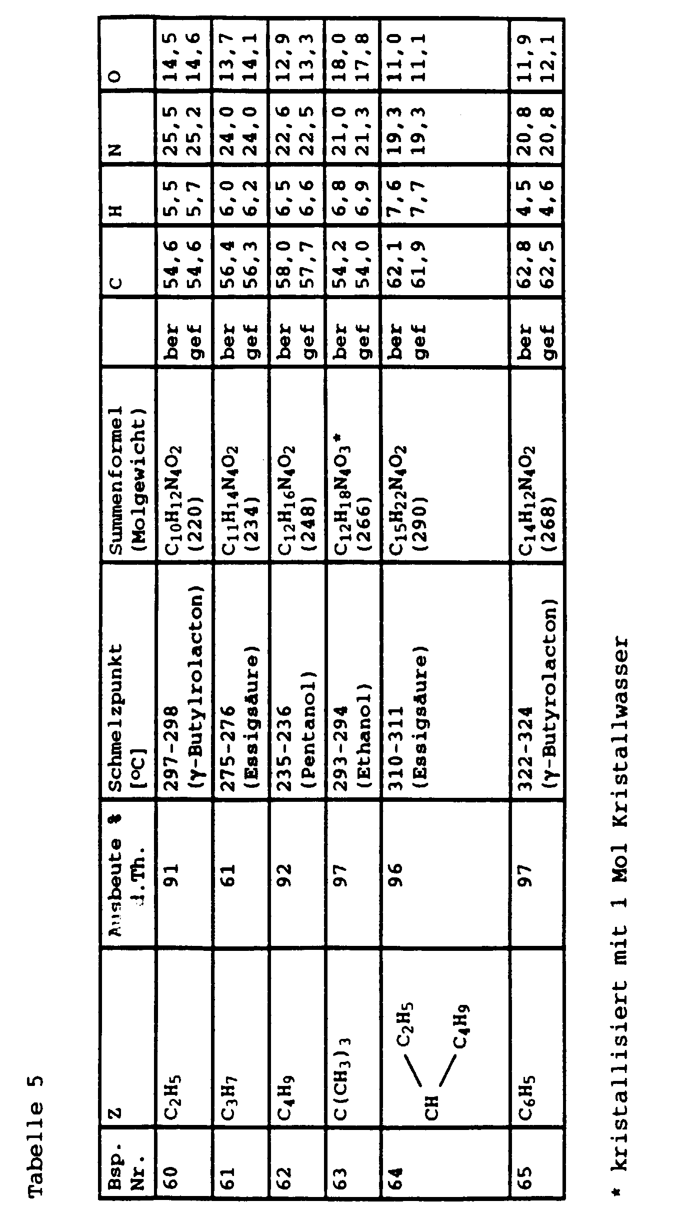

Allgemeine Vorschrift zum Ringschluß der acylierten Hydrazinopyridine:

1 mol acyliertes Hydrazinopyridin wird in der 2,5-fachen Menge N-Methylpyrrolidon bei 80 bis 100°C gerührt und anteilweise mit 1,1 mol Phosphorpentoxid versetzt. Man rührt 4 Stunden bei 125°C, fällt auf Wasser, saugt ab, wäscht mit Wasser neutral und trocknet bei 100°C.

In der folgenden Tabelle 6 sind die nach obiger Vorschrift erhaltenen Verbindungen der Formel 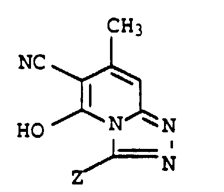 aufgeführt.

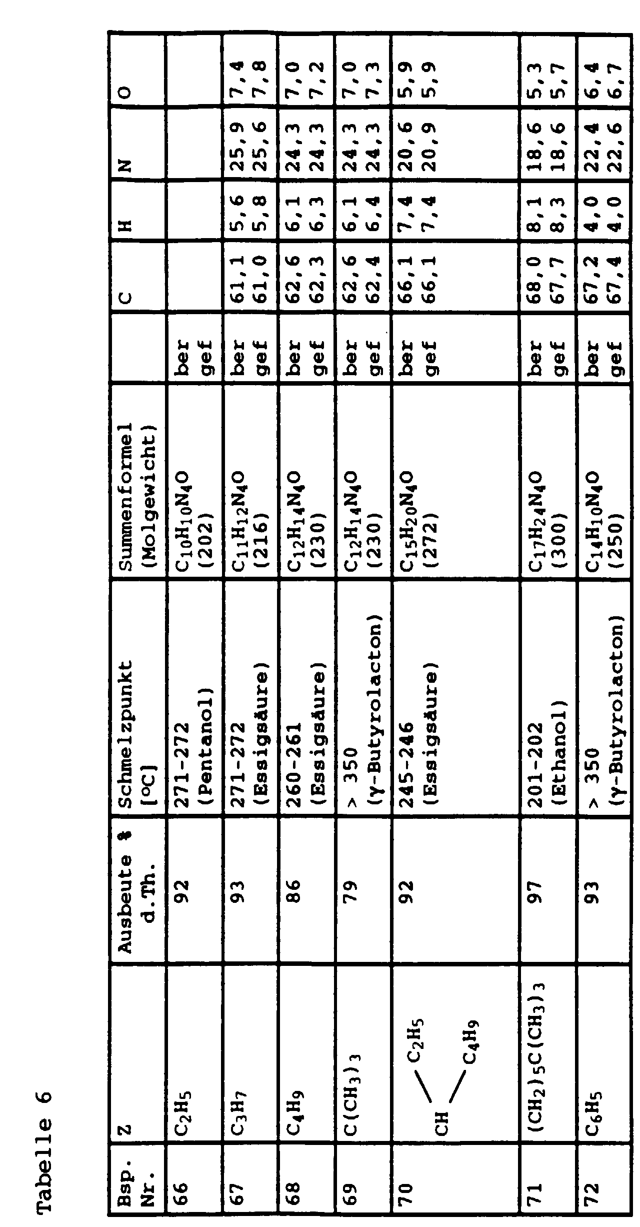

### Beispiel 73

In 1000 ml Pyridin wurden 164 g 2-Hydroxy-3-cyano-4-methyl-6-hydrazinopyridin eingetragen und bei 70°C gerührt. Dazu goß man anteilweise 104 g Bernsteinsäureanhydrid und erhitzte anschließend 6 Stunden unter Rückfluß. Dann wurde das Reaktionsgemisch auf Eis und überschüssiger Salzsäure gefällt, das ausgefallene Produkt abgesaugt und mit Wasser gewaschen. Nach dem Trocknen erhielt man 210 g der Verbindung der Formel 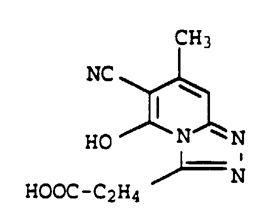 Die Verbindung ist löslich in wäßrigem Ammoniak. Eine aus γ-Butyrolacton/Essigsaure umkristallisierte Probe schmilzt bei 329 bis 330°C und zeigt folgende Analyse:

| C₁₁H₁₀N₄O₃ (246) | | | | |
|---|---|---|---|---|
| ber | C 53,7 | H 4,1 | N 22,7 | O 19,5 |
| gef | C 53,9 | H 4,5 | N 22,4 | O 19,5 |

### Beispiel 74

Man ersetzte in Beispiel 74 Bernsteinsäureanhydrid durch 250 g Isobuttersäurechlorid und verfuhr ansonsten, wie in Beispiel 73 beschrieben. Man erhielt 179 g der Verbindung der Formel 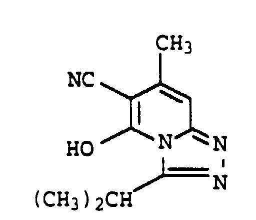 Schmelzpunkt 345 bis 347°C (aus Ethanol)

| C₁₁H₁₂N₄O (216) | | | | |
|---|---|---|---|---|
| ber | C 61,1 | H 5,6 | N 25,9 | O 7,4 |
| gef | C 60,8 | H 5,8 | N 26,0 | O 7,6 |

### Beispiel 75

In 100 ml Chloroform wurden 12 g N,N-Dimethylformamid und 27,2 g der nach Beispiel 70 erhaltenen Verbindung eingetragen und gerührt. Man tropfte 25 g Phosphoroxidtrichlorid zu und erhitzte 6 Stunden unter Rückfluß. Dann wurde das Chloroform mit Wasserdampf abdestilliert und die verbleibende Kristallsuspension abgesaugt.

Man erhielt nach dem Trocknen bei 100°C 28,4 g der Verbindung der Formel 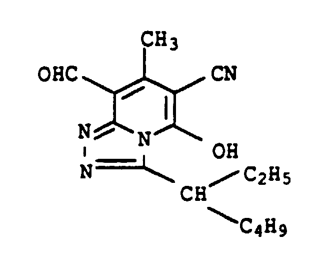 in Form leicht gelbstichiger Kristalle; Schmelzpunkt 183 bis 184°C (aus Ethanol).

| C₁₆H₂₀N₄O₂ (300) | | | | |
|---|---|---|---|---|
| ber | C 64,0 | H 6,7 | N 18,7 | O 10,7 |
| gef | C 63,7 | H 6,8 | N 18,6 | O 11,0 |

### Beispiel 76

In 100 ml Pyridin wurden 30 g Schwefelkohlenstoff und 16,4 g 2-Hydroxy-3-cyano-4-methyl-6-hydrazinopyridin eingetragen und 6 Stunden bei 60°C gerührt. Man verdünnte die Kristallsuspension mit 100 ml Methanol, saugte ab und wusch mit Wasser. Nach dem Trocknen erhielt man 24,9 g der Verbindung der Formel 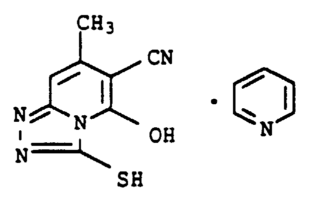 mit einem Schmelzpunkt von 220 bis 221°C

| C₁₃H₁₁N₅OS (285) | | |
|---|---|---|
| ber | N 24,5 | S 11,2 |
| gef | N 24,2 | S 10,9 |

### Beispiel 77

Zu einer Mischung aus 200 ml Ethanol, 20 g Schwefelkohlenstoff und 16,4 g 2-Hydroxy-3-cyano-4-methyl-6-hydrazinopyridin wurden 25 g 20 gew.%ige Natronlauge gegossen. Nach 2-stündigem Erhitzen unter Rückfluß fügte man 100 ml Wasser und 20 g n-Butylbromid zu und rührte weitere 2 Stunden bei 50°C. Man stellte dann mit Essigsäure neutral, saugte ab und wusch mit Wasser. Nach dem Trocknen erhielt man 23,9 g der Verbindung der Formel 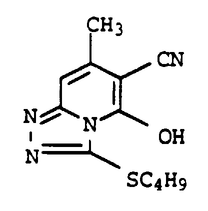 in Form geblichen Kristalls; Schmelzpunkt 277 bis 278°C (aus Pentanol).

| C₁₂H₁₄N₄OS (262) | | | | | |
|---|---|---|---|---|---|
| ber | C 54,9 | H 5,4 | N 21,4 | O 6,1 | S 12,2 |
| gef | C 54,9 | H 5,5 | N 21,5 | O 6,3 | S 12,0 |

Analog Beispiel 77 werden die in der folgenden Tabelle 7 aufgeführten Verbindungen der Formel 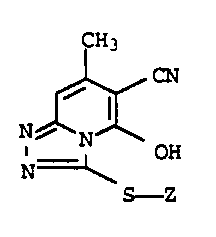 erhalten.

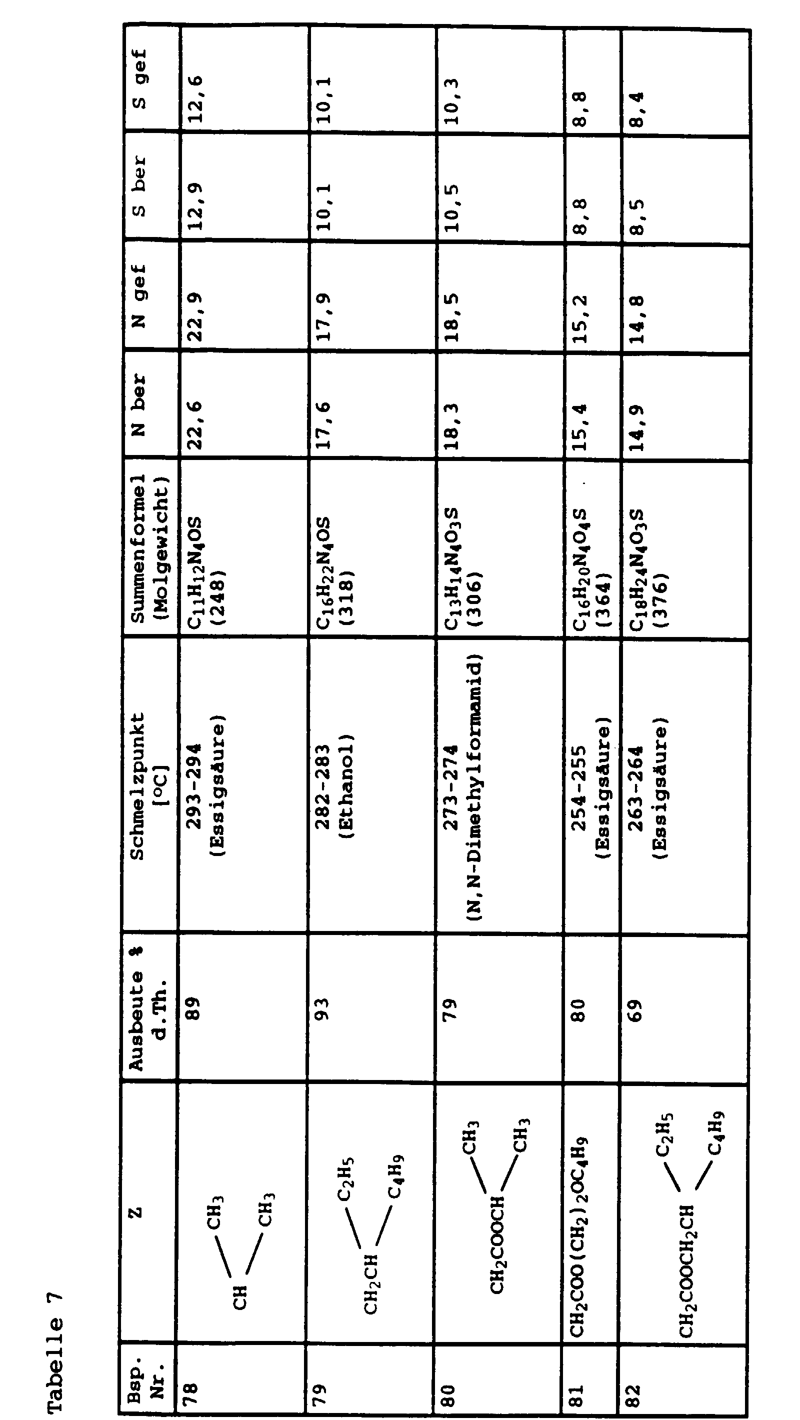

## Patentansprüche

1. Triazolopyridine der Formel I 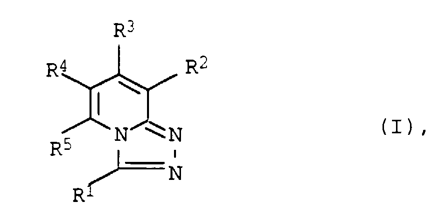 in der
R¹ C₁-C₂₀-Alkyl, das gegebenenfalls durch Phenyl, C₁-C₄-Alkylphenyl, C₁-C₄-Alkoxyphenyl, Halogenphenyl, Nitrophenyl, Carboxylphenyl, Phenoxy, C₁-C₄-Alkylphenoxy, C₁-C₄-Alkoxyphenoxy, Halogenphenoxy, Nitrophenoxy, Carboxylphenoxy, Carboxyl oder C₁-C₂₀-Alkoxycarbonyl, dessen Alkylkette gegebenenfalls durch 1 bis 4 Sauerstoffatome in Etherfunktion unterbrochen ist und durch Phenyl oder Phenoxy substituiert sein kann, substituiert ist und durch 1 bis 4 Sauerstoffatome in Etherfunktion unterbrochen sein kann, gegebenenfalls durch C₁-C₄-Alkyl, C₁-C₄-Alkoxy, Halogen, Nitro oder Carboxyl substituiertes Phenyl, Mercapto oder gegebenenfalls durch Phenyl, C₁-C₄-Alkylphenyl, C₁-C₄-Alkoxyphenyl, Halogenphenyl, Nitrophenyl, Carboxylphenyl, Phenoxy, C₁-C₄-Alkylphenoxy, C₁-C₄-Alkoxyphenoxy, Halogenphenoxy, Nitrophenoxy, Carboxylphenoxy, Carboxyl oder C₁-C₂₀-Alkoxycarbonyl, dessen Alkylkette gegebenenfalls durch 1 bis 4 Sauerstoffatome in Etherfunktion unterbrochen ist und durch Phenyl oder Phenoxy substituiert sein kann, substituiertes C₁-C₂₀-Alkylthio,
R² Wasserstoff, Formyl, Nitroso, Cyano, C₁-C₄-Alkoxymethyl oder einen Rest der Formel CH₂-NL¹L², wobei L¹ und L² gleich oder verschieden sind und unabhängig voneinander jeweils für Wasserstoff oder C₁-C₄-Alkyl, das gegebenenfalls durch C₁-C₄-Alkylimino unterbrochen ist, oder zusammen mit dem sie verbindenden Stickstoffatom für einen 5- oder 6-gliedrigen gesättigten heterocyclischen Rest stehen,
R³ C₁-C₄-Alkyl, das gegebenenfalls durch ein Sauerstoffatom in Etherfunktion unterbrochen ist, C₁-C₄-Alkoxycarbonyl oder Phenyl,
R⁴ Wasserstoff, Cyano, Carbamoyl, Carboxyl oder C₁-C₄-Alkoxycarbonyl und
R⁵ Hydroxy, Mercapto, Halogen, den Rest -NL¹L², wobei L¹ und L² jeweils die obengenannte Bedeutung besitzen, oder den Rest einer CH-aciden Verbindung bedeuten, wobei als CH-acide Verbindungen Nitromethan, Nitroethan oder Verbindungen der Formeln VII bis XII 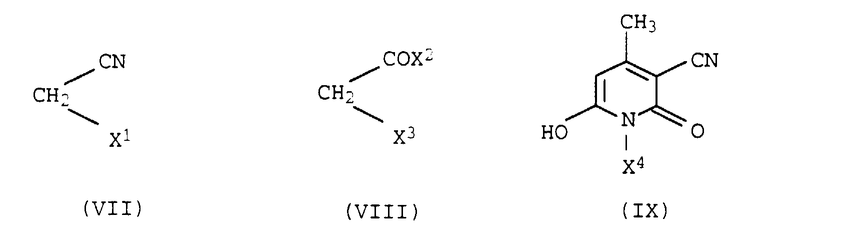 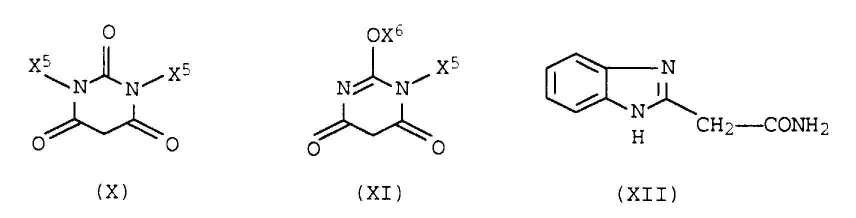
in Betracht kommen, wobei
X¹ Cyano, Nitro, C₁-C₄-Alkanoyl, gegebenenfalls durch C₁-C₄-Alkyl, C₁-C₄-Alkoxy oder Halogen substituiertes Benzoyl, C₁-C₄-Alkylsulfonyl, gegebenenfalls durch C₁-C₄-Alkyl, C₁-C₄-Alkoxy oder Halogen substituiertes Phenylsulfonyl, Carboxyl, C₁-C₄-Alkoxycarbonyl, Phenoxycarbonyl, Carbamoyl, C₁-C₄-Mono- oder Dialkylcarbamoyl, gegebenenfalls durch C₁-C₄-Alkyl, C₁-C₄-Alkoxy oder Halogen substituiertes Phenylcarbamoyl, gegebenenfalls durch C₁-C₄-Alkyl, C₁-C₄-Alkoxy, Halogen oder Nitro substituiertes Phenyl, Benzthiazol-2-yl, Benzimidazol-2-yl, 5-Phenyl-1,3,4-thiadiazol-2-yl oder 2-Hydroxychinoxalin-3-yl,
X² C₁-C₄-Alkyl oder C₁-C₄-Alkoxy,
X³ C₁-C₄-Alkoxycarbonyl, Phenylcarbamoyl oder Benzimidazol-2-yl,
X⁴ Wasserstoff oder C₁-C₆-Alkyl,
X⁵ Wasserstoff, C₁-C₄-Alkyl oder Phenyl und
X⁶ C₁-C₄-Alkyl bedeuten.

2. Triazolopyridine nach Anspruch 1, dadurch gekennzeichnet, daß einer der beiden Reste R² und R⁴ Wasserstoff und der andere Cyano bedeutet.

3. Triazolopyridine nach Anspruch 1, dadurch gekennzeichnet, daß R² Cyano bedeutet.

4. Triazolopyridine nach Anspruch 1, dadurch gekennzeichnet, daß R² Cyano und R⁴ Wasserstoff bedeuten.

5. Triazolopyridine nach Anspruch 1, dadurch gekennzeichnet, daß R³ C₁-C₄-Alkyl bedeutet.

6. Triazolopyridine nach Anspruch 1, dadurch gekennzeichnet, daß R¹ C₁-C₁₃-Alkyl oder Phenyl bedeutet.

7. Verfahren zur Herstellung von Triazolopyridinen gemäß Anspruch 1, die der Formel Ia 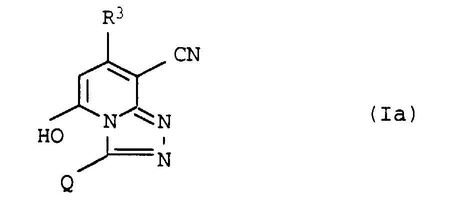 gehorchen, in der
Q C₁-C₂₀-Alkyl, das gegebenenfalls durch Phenyl, C₁-C₄-Alkylphenyl, C₁-C₄-Alkoxyphenyl, Halogenphenyl, Nitrophenyl, Carboxylphenyl, Phenoxy, C₁-C₄-Alkylphenoxy, C₁-C₄-Alkoxyphenoxy, Halogenphenoxy, Nitrophenoxy, Carboxylphenoxy, Carboxyl oder C₁-C₂₀-Alkoxycarbonyl, dessen Alkylkette gegebenenfalls durch 1 bis 4 Sauerstoffatome in Etherfunktion unterbrochen ist und durch Phenyl oder Phenoxy substituiert sein kann, substituiert ist und durch 1 bis 4 Sauerstoffatome in Etherfunktion unterbrochen sein kann, oder gegebenenfalls durch C₁-C₄-Alkyl, C₁-C₄-Alkoxy, Halogen, Nitro oder Carboxyl substituiertes Phenyl bedeutet und
R³ die in Anspruch 1 genannte Bedeutung besitzt,
dadurch gekennzeichnet, daß man eine Dicarbonylverbindung der Formel II
R³-CO-CH₂-COOW (II),
in der W C₁-C₄-Alkyl bedeutet und R³ die in Anspruch 1 genannte Bedeutung besitzt, mit einem Cyanomethyltriazol der Formel III 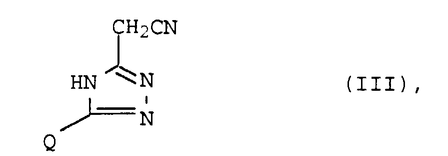 in der Q die obengenannte Bedeutung besitzt, in Gegenwart einer Base und eines Lösungsmittels bei einer Temperatur von 50 bis 150°C umsetzt.

8. Verfahren zur Herstellung von Triazolopyridinen gemäß Anspruch 1, die der Formel Ib 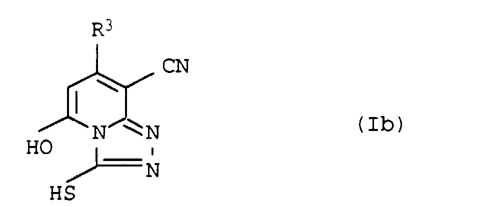 gehorchen, in der R³ die in Anspruch 1 genannte Bedeutung besitzt, dadurch gekennzeichnet, daß man ein Hydrazinopyridin der Formel IV 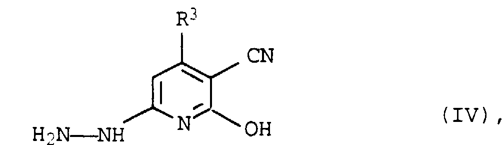 in der R³ die obengenannte Bedeutung besitzt, mit Schwefelkohlenstoff behandelt.

9. Verfahren zur Herstellung von Triazolopyridinen gemäß Anspruch 1, die der Formel Ic 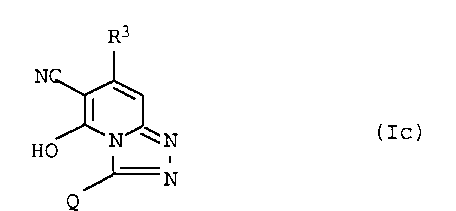 gehorchen, in der
Q C₁-C₂₀-Alkyl, das gegebenenfalls durch Phenyl, C₁-C₄-Alkylphenyl, C₁-C₄-Alkoxyphenyl, Halogenphenyl, Nitrophenyl, Carboxylphenyl, Phenoxy, C₁-C₄-Alkylphenoxy, C₁-C₄-Alkoxyphenoxy, Halogenphenoxy, Nitrophenoxy, Carboxylphenoxy, Carboxyl oder C₁-C₂₀-Alkoxycarbonyl, dessen Alkylkette gegebenenfalls durch 1 bis 4 Sauerstoffatome in Etherfunktion unterbrochen ist und durch Phenyl oder Phenoxy substituiert sein kann, substituiert ist und durch 1 bis 4 Sauerstoffatome in Etherfunktion unterbrochen sein kann, oder gegebenenfalls durch C₁-C₄-Alkyl, C₁-C₄-Alkoxy, Halogen, Nitro oder Carboxyl substituiertes Phenyl bedeutet und
R³ die in Anspruch 1 genannte Bedeutung besitzt,
dadurch gekennzeichnet, daß man ein Hydrazinopyridin der Formel IV 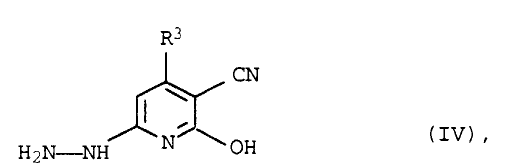 in der R³ die in Anspruch 1 genannte Bedeutung besitzt, mit einem Carbonsäurederivat der Formel V
Q-CO-Y (V),
in der Y Chlor, Brom oder der Rest O-CO-Q bedeutet und Q jeweils die obengenannte Bedeutung besitzt, acyliert und die resultierende Acylverbindung der Formel VI 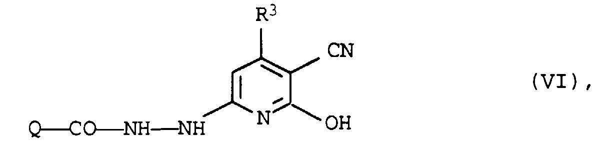 in der Q und R³ jeweils die obengenannte Bedeutung besitzen, unter Ringschluß dehydratisiert.

## Claims

1. A triazolopyridine of the formula I 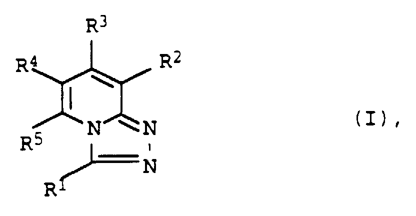 where
R¹ is C₁-C₂₀-alkyl which is unsubstituted or substituted by phenyl, C₁-C₄-alkylphenyl, C₁-C₄-alkoxyphenyl, halophenyl, nitrophenyl, carboxyphenyl, phenoxy, C₁-C₄-alkylphenoxy, C₁-C₄-alkoxyphenoxy, halophenoxy, nitrophenoxy, carboxyphenoxy, carboxyl or C₁-C₂₀-alkoxycarbonyl whose alkyl chain is uninterrupted or interrupted by 1 to 4 oxygen atoms in ether form and can be substituted by phenyl or phenoxy, and can be interrupted by 1 to 4 oxygen atoms in ether form, or is phenyl which is unsubstituted or substituted by C₁-C₄-alkyl, C₁-C₄-alkoxy, halogen, nitro or carboxyl, mercapto or C₁-C₂₀-alkylthio which is unsubstituted or substituted by phenyl, C₁-C₄-alkylphenyl, C₁-C₄-alkoxyphenyl, halophenyl, nitrophenyl, carboxyphenyl, phenoxy, C₁-C₄-alkylphenoxy, C₁-C₄-alkoxyphenoxy, halophenoxy, nitrophenoxy, carboxyphenoxy, carboxyl or C₁-C₂₀-alkoxycarbonyl whose alkyl chain is uninterrupted or interrupted by 1 to 4 oxygen atoms in ether form and can be substituted by phenyl or phenoxy,
R² is hydrogen, formyl, nitroso, cyano, C₁-C₄-alkoxymethyl or a radical of the formula CH₂-NL¹L², where L¹ and L² are identical or different and independently of one another in each case are hydrogen or C₁-C₄-alkyl which may be interrupted by C₁-C₄-alkylimino, or together with the nitrogen bonding them are a 5- or 6-membered saturated heterocyclic radical,
R³ is C₁-C₄-alkyl which may be interrupted by an ether oxygen atom, or is C₁-C₄-alkoxycarbonyl or phenyl,
R⁴ is hydrogen, cyano, carbamoyl, carboxyl or C₁-C₄-alkoxycarbonyl and
R⁵ is hydroxyl, mercapto, halogen, the radical -NL¹L², where L¹ and L² in each case have the abovementioned meanings, or the radical of a CH-acidic compound, suitable CH-acidic compounds being nitromethane, nitroethane or compounds of the formulae VII to XII 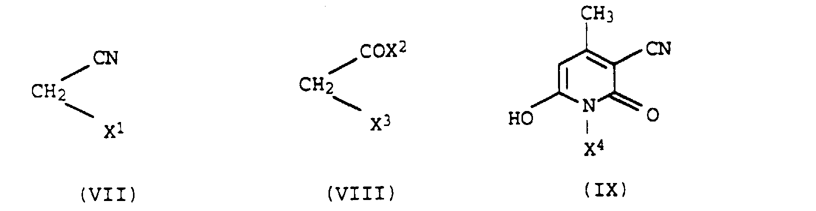 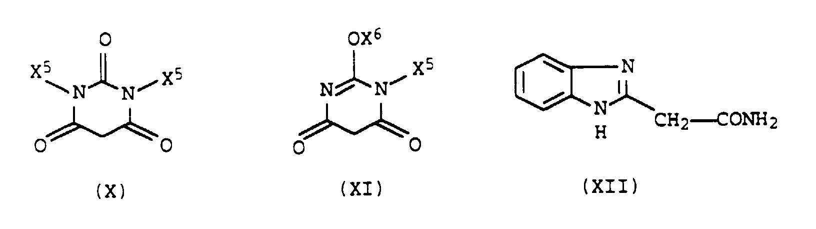
where
X¹ is cyano, nitro, C₁-C₄-alkanoyl, benzoyl which is unsubstituted or substituted by C₁-C₄-alkyl, C₁-C₄-alkoxy or halogen, C₁-C₄-alkylsulfonyl, or phenylsulfonyl, carboxyl, C₁-C₄-alkoxycarbonyl, phenoxycarbonyl, carbamoyl, C₁-C₄-monoalkylcarbamoyl or C₁-C₄-dialkylcarbamoyl, each of which is unsubstituted or substituted by C₁-C₄-alkyl, C₁-C₄-alkoxy or halogen, or phenylcarbamoyl which is unsubstituted or substituted by C₁-C₄-alkyl, C₁-C₄-alkoxy or halogen, or phenyl, benzothiazol-2-yl, benzimidazol-2-yl, 5-phenyl-1,3,4-thiadiazol-2-yl or 2-hydroxyquinoxalin-3-yl, each of which is unsubstituted or substituted by C₁-C₄-alkyl, C₁-C₄-alkoxy, halogen or nitro,
X² is C₁-C₄-alkyl or C₁-C₄-alkoxy,
X³ is C₁-C₄-alkoxycarbonyl, phenylcarbamoyl or benzimidazol-2-yl,
X⁴ is hydrogen or C₁-C₆-alkyl,
X⁵ is hydrogen, C₁-C₄-alkyl or phenyl and
X⁶ is C₁-C₄-alkyl.

2. A triazolopyridine as claimed in claim 1, wherein one of the two radicals R² and R⁴ is hydrogen and the other is cyano.

3. A triazolopyridine as claimed in claim 1, wherein R² is cyano.

4. A triazolopyridine as claimed in claim 1, wherein R² is cyano and R⁴ is hydrogen.

5. A triazolopyridine as claimed in claim 1, wherein R³ is C₁-C₄-alkyl.

6. A triazolopyridine as claimed in claim 1, wherein R¹ is C₁-C₁₃-alkyl or phenyl.

7. A process for preparing triazolopyridines as claimed in claim 1, which correspond to the formula Ia 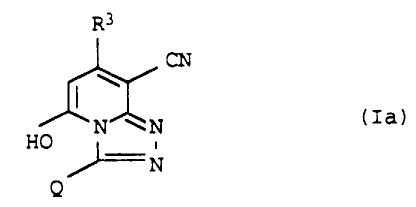 where
Q is C₁-C₂₀-alkyl which is unsubstituted or substituted by phenyl, C₁-C₄-alkylphenyl, C₁-C₄-alkoxyphenyl, halophenyl, nitrophenyl, carboxyphenyl, phenoxy, C₁-C₄-alkylphenoxy, C₁-C₄-alkoxyphenoxy, halophenoxy, nitrophenoxy, carboxyphenoxy, carboxyl or C₁-C₂₀-alkylcarbonyl whose alkyl chain is uninterrupted or interrupted by 1 to 4 oxygen atoms in ether form and can be substituted by phenyl or phenoxy, and can be interrupted by 1 to 4 oxygen atoms in ether form, or is phenyl which is unsubstituted or substituted by C₁-C₄-alkyl, C₁-C₄-alkoxy, halogen, nitro or carboxyl and
R³ has the meanings mentioned in claim 1,
which comprises reacting a dicarbonyl compound of the formula II
R³-CO-CH₂-COOW (II),
where W is C₁-C₄-alkyl and R³ has the meanings mentioned in claim 1, with a cyanomethyltriazole of the formula III 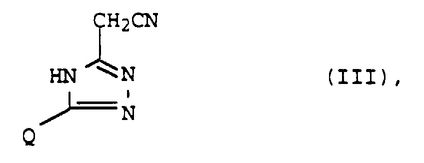 where Q has the abovementioned meanings, in the presence of a base and of a solvent at from 50 to 150°C.

8. A process for preparing triazolopyridines as claimed in claim 1, which correspond to the formula Ib 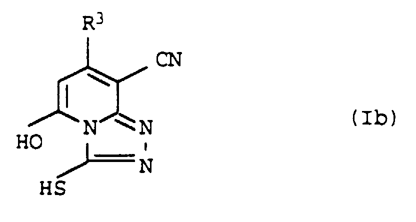 where R³ has the meanings mentioned in claim 1, which comprises treating a hydrazinopyridine of the formula IV 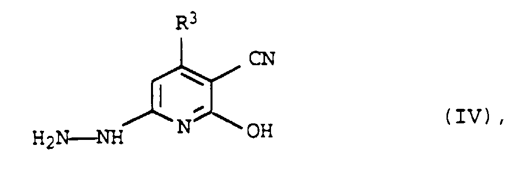 where R³ has the abovementioned meanings, with carbon disulfide.

9. A process for preparing triazolopyridines as claimed in claim 1, which correspond to the formula Ic 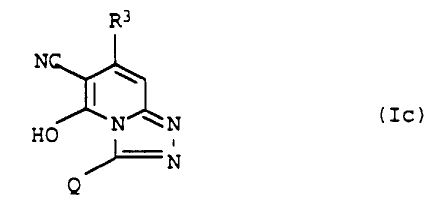 where
Q is C₁-C₂₀-alkyl which is unsubstituted or substituted by phenyl, C₁-C₄-alkylphenyl, C₁-C₄-alkoxyphenyl, halophenyl, nitrophenyl, carboxyphenyl, phenoxy, C₁-C₄-alkylphenoxy, C₁-C₄-alkoxyphenoxy, halophenoxy, nitrophenoxy, carboxyphenoxy, carboxyl or C₁-C₂₀-alkylcarbonyl whose alkyl chain is uninterrupted or interrupted by 1 to 4 oxygen atoms in ether form and can be substituted by phenyl or phenoxy, and can be interrupted by 1 to 4 oxygen atoms in ether form, or is phenyl which is unsubstituted or substituted by C₁-C₄-alkyl, C₁-C₄-alkoxy, halogen, nitro or carboxyl and
R³ has the meanings mentioned in claim 1,
which comprises acylating a hydrazinopyridine of the formula IV 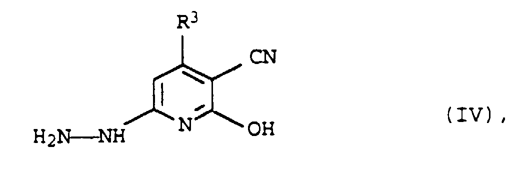 where R³ has the meanings mentioned in claim 1, with a carboxylic acid derivative of the formula V
Q-CO-Y (V),
where Y is chlorine, bromine or the radical O-CO-Q and Q in each case has the abovementioned meanings, and dehydrating the resulting acyl compound of the formula VI 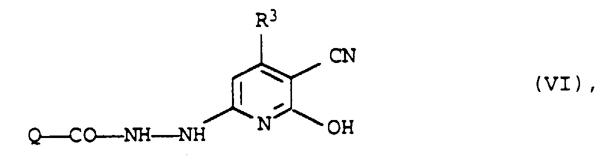 where Q and R³ in each case have the abovementioned meanings, with ring closure.

## Revendications

1. Triazolopyridines de formule I 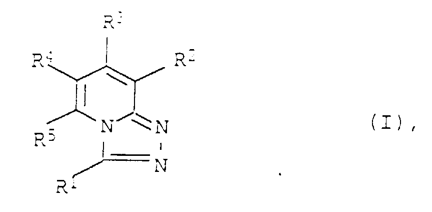 dans laquelle
R¹ représente un groupement alkyle en C₁-C₂₀, éventuellement substitué par un groupement phényle, alkylphényle en C₁-C₄, alkoxyphényle en C₁-C₄, halogénophényle, nitrophényle, carboxylphényle, phénoxy, alkylphénoxy en C₁-C₄, alkoxyphénoxy en C₁-C₄, halogénophénoxy, nitrophénoxy, carboxylphénoxy, carboxyle ou alcoxycarbonyle en C₁-C₂₀, dont la chaîne alkyle peut éventuellement être interrompue par 1 à 4 atomes d'oxygène en fonction éther et peut être substituée par un groupement phényle ou phénoxy, et qui peut être interrompu par 1 à 4 atomes d'oxygène en fonction éther, un groupement phényle éventuellement substitué par un groupement alkyle en C₁-C₄, alcoxy en C₁-C₄, un atome d'halogène, un groupement nitro ou carboxyle, un groupement mercapto ou un groupement alkylthio en C₁-C₂₀ éventuellement substitué par un groupement phényle, alkylphényle en C₁-C₄, alcoxyphényle en C₁-C₄, halgénophényle, nitrophényle, carboxylphényle, phénoxy, alkylphénoxy en C₁-C₄, alkoxyphénoxy en C₁-C₄, halogénophénoxy, nitrophénoxy, carboxylphénoxy, carboxyle ou alcoxycarbonyle en C₁-C₂₀, dont la chaîne alkyle peut éventuellement être interrompue par 1 à 4 atomes d'oxygène en fonction éther et peut être substituée par un groupement phényle ou phénoxy,
R² représente un atome d'hydrogène, un groupement formyle, nitroso, cyano, alcoxyméthyle en C₁-C₄ ou un groupement de formule CH₂-NL¹L², où L¹ et L² sont identiques ou différents et sont mis chacun indépendamment l'un de l'autre pour un atome d'hydrogène ou un groupement alkyle en C₁-C₄, qui est éventuellement interrompu par un groupement alkylamino en C₁-C₄, ou bien pour un groupement hétérocyclique saturé à 5 ou 6 maillons formé avec l'atome d'azote qui les relie,
R³ représente un groupement alkyle en C₁-C₄, qui est éventuellement interrompu par un atome d'oxygène en fonction éther, un groupement alcoxycarbonyle en C₁-C₄ ou phényle,
R⁴ représente un atome d'hydrogène, un groupement cyano, carbamoyle, carboxyle ou alcoxycarbonyle en C1-C4 et
R⁵ représente un groupement hydroxy, mercapto, un atome d'halogène, un groupement -NL¹L², où L¹ et L² prennent chacun la signification ci-dessus, ou représentent le reste d'un composé à CH acide, où l'on prend en compte en tant que composés à CH acide, le nitrométhane, le nitroéthane ou des composés de formules VII à XII 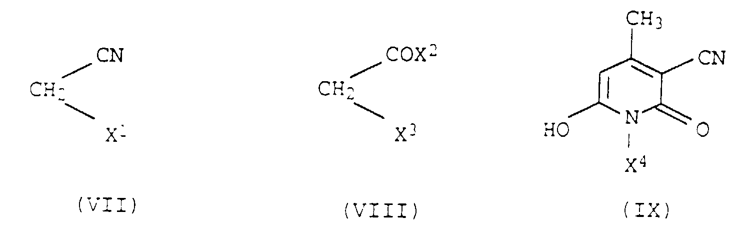 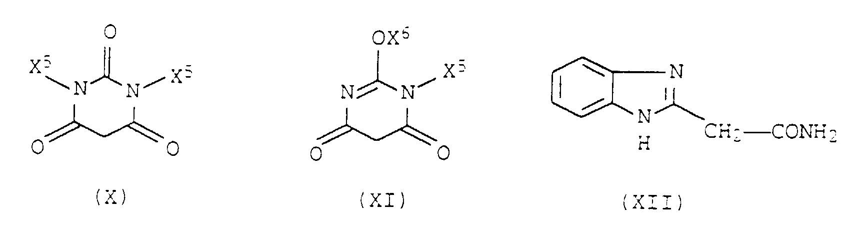
où,
X¹ représente un groupement cyano, nitro, alcanoyle en C₁-C₄, un groupement benzoyle éventuellement substitué par un groupement alkyle en C₁-C₄, alcoxy en C₁-C₄ ou un atome d'halogène, un groupement alkylsulfonyle en C₁-C₄, un groupement phénylsulfonyle éventuellement substitué par un groupement alkyle en C₁-C₄, alcoxy en C₁-C₄ ou un atome d'halogène, un groupement carboxyle, alcoxycarbonyle en C₁-C₄, phénoxycarbonyle, carbamoyle, mono- ou dialkylcarbamoyle en C₁-C₄, un groupement phénylcarbamoyle éventuellement substitué par un groupement alkyle en C₁-C₄, alcoxy en C₁-C₄ ou un atome d'halogène, un groupement phényle éventuellement substitué par un groupement alkyle en C₁-C₄, alcoxy en C₁-C₄, un atome d'halogène ou un groupement nitro, un groupement benzothiazol-2-yle, benzimidazol-2-yle, 5-phényl-1,3,4-thiadiazol-2-yle ou hydroxyquinoxalin-3-yle,
X² représente un groupement alkyle en C₁-C₄ ou alcoxy en C₁-C₄,
X³ représente un groupement alcoxycarbonyle en C₁-C₄, phénylcarbamoyle ou benzimidazol-2-yle,
X⁴ représente un atome d'hydrogène ou un groupement alkyle en C₁-C₆,
X⁵ représente un atome d'hydrogène, un groupement alkyle en C₁-C₄ ou phényle et
X⁶ représente un groupement alkyle en C₁-C₄.

2. Triazolopyridines selon la revendication 1, caractérisé en ce que l'un des deux groupements R² et R⁴ représente un atome d'hydrogène et l'autre un groupement cyano.

3. Triazolopyridines selon la revendication 1, caractérisé en ce que R² représente un groupement cyano.

4. Triazolopyridines selon la revendication 1, caractérisé en ce que R² représente un groupement cyano et R⁴ un atome d'hydrogène.

5. Triazolopyridines selon la revendication 1, caractérisé en ce que R³ représente un groupement alkyle en C₁-C₄.

6. Triazolopyridines selon la revendication 1, caractérisé en ce que R¹ représente un groupement alkyle en C₁-C₁₃ ou phényle.

7. Procédé de fabrication de triazolopyridines selon la revendication 1, qui suivent la formule Ia 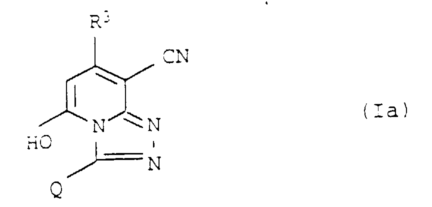 dans laquelle
Q représente un groupement alkyle en C₁-C₂₀, qui est éventuellement substitué par un groupement phényle, alkylphényle en C₁-C₄, alcoxyphényle en C₁-C₄, halogénophényle, nitrophényle, carboxylphényle, phénoxy, alkylphénoxy en C₁-C₄, alcoxyphénoxy en C₁-C₄, halogénophénoxy, nitrophénoxy, carboxylphénoxy, carboxyle ou alcoxycarbonyle en C₁-C₂₀, dont la chaîne alkyle est éventuellement interrompue par 1 à 4 atomes d'oxygène en fonction éther et qui peut être substituée par un groupement phényle ou phénoxy, et qui peut être interrompu par 1 à 4 atomes d'oxygène en fonction éther, ou un groupement phényle éventuellement substitué par un groupement alkyle en C₁-C₄, alcoxy en C₁-C₄, un atome d'halogène, un groupement nitro ou carboxyle et
R³ prend la signification mentionnée dans la revendication 1,
caractérisé en ce que l'on fait réagir un composé dicarbonylé de formule II
R³-CO-CH₂-COOW (II),
dans laquelle W représente un groupement alkyle en C₁-C₄ et R³ prend la signification mentionnée dans la revendication 1, avec un cyanométhyltriazole de formule III 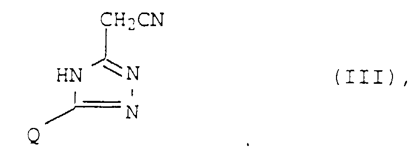 dans laquelle Q prend la signification mentionnée ci-dessus, en présence d'une base et d'un solvant à une température de 50 à 150°C.

8. Procédé de fabrication de triazolopyridines selon la revendication 1, qui suivent la formule Ib 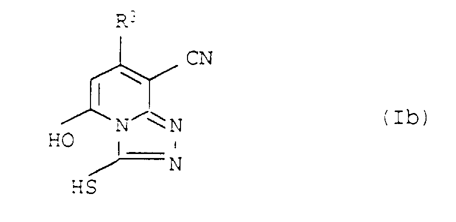 dans laquelle R³ prend la signification mentionnée dans la revendication 1, caractérisé en ce que l'on traite une hydrazinopyridine de formule IV 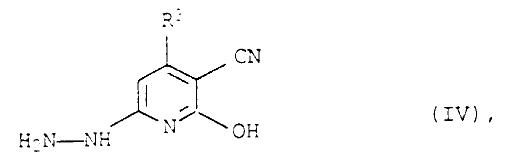 dans laquelle R³ prend la signification susmentionnée, avec du sulfure de carbone.

9. Procédé de fabrication de triazolopyridines selon la revendication 1, qui suivent la formule Ic 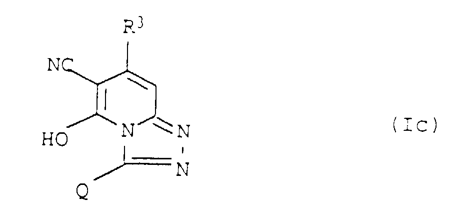 dans laquelle
Q représente un groupement alkyle en C₁-C₂₀, qui est éventuellement substitué par un groupement phényle, alkylphényle en C₁-C₄, alcoxyphényle en C₁-C₄, halogénophényle, nitrophényle, carboxylphényle, phénoxy, alkylphénoxy en C₁-C₄, alcoxyphénoxy en C₁-C₄, halogénophénoxy, nitrophénoxy, carboxylphénoxy, carboxyle ou alcoxycarbonyle en C₁-C₂₀, dont la chaîne alkyle est éventuellement interrompue par 1 à 4 atomes d'oxygène en fonction éther et qui peut être substituée par un groupement phényle ou phénoxy, et qui peut être interrompu par 1 à 4 atomes d'oxygène en fonction éther, ou bien un groupement phényle éventuellement substitué par un groupement alkyle en C₁-C₄, alcoxy en C₁-C₄, un atome d'halogène, un groupement nitro ou carboxyle et
R³ prend la signification mentionnée dans la revendication 1,
caractérisé en ce que l'on acyle une hydrazinopyridine de formule IV 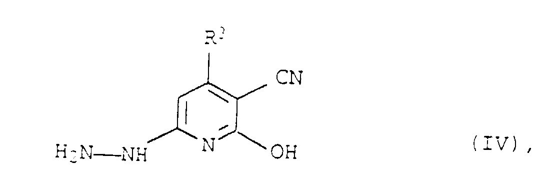 dans laquelle R³ prend la signification mentionnée dans la revendication 1, avec un dérivé d'acide carboxylique de formule V
Q-CO-Y (V),
dans laquelle Y représente un atome de chlore, de brome ou un groupement O-CO-Q et Q prend à chaque fois la signification mentionnée ci-dessus, et le composé acylé résultant de formule VI 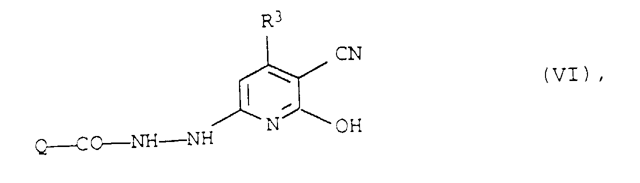 dans laquelle Q et R³ prennent chacun la signification susmentionnée, est déshydraté par fermeture du cycle.
